(19) Europäisches Patentamt
European Patent Office
Office européen des brevets

(11) EP 2 010 294 B1

(12) **EUROPÄISCHE PATENTSCHRIFT**

(45) Veröffentlichungstag und Bekanntmachung des Hinweises auf die Patenterteilung:
**02.12.2015 Patentblatt 2015/49**

(21) Anmeldenummer: **07724050.5**

(22) Anmeldetag: **05.04.2007**

(51) Int Cl.:
***A61Q 19/02*** ^(2006.01) ***A61Q 17/04*** ^(2006.01)
***A61K 8/34*** ^(2006.01) ***A61K 8/35*** ^(2006.01)
***A61K 8/368*** ^(2006.01) ***A61K 8/41*** ^(2006.01)
***A23L 3/3481*** ^(2006.01) ***A23L 3/3526*** ^(2006.01)

(86) Internationale Anmeldenummer:
**PCT/EP2007/003109**

(87) Internationale Veröffentlichungsnummer:
**WO 2007/121845 (01.11.2007 Gazette 2007/44)**

(54) **ANTIOXIDANTIEN**

ANTIOXIDANTS

ANTIOXYDANTS

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **25.04.2006 DE 102006019044**

(43) Veröffentlichungstag der Anmeldung:
**07.01.2009 Patentblatt 2009/02**

(73) Patentinhaber: **Merck Patent GmbH**
**64293 Darmstadt (DE)**

(72) Erfinder:
- **RUDOLPH, Thomas**
  **64291 Darmstadt (DE)**
- **BUCHHOLZ, Herwig**
  **60599 Frankfurt (DE)**

(56) Entgegenhaltungen:
**WO-A-2004/046334** **WO-A-2005/004630**
**GB-A- 1 345 988** **JP-A- 1 151 530**
**JP-A- 2 160 737** **JP-A- 56 022 711**
**US-A- 6 066 327**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 010 294 B1

Printed by Jouve, 75001 PARIS (FR)

## Beschreibung

Die vorliegende Erfindung betrifft die Verwendung von Verbindungen als Antioxidans bzw. zum Produktschutz bzw. zur Pigmentierungskontrolle, entsprechende neue Verbindungen und Zubereitungen, sowie entsprechende Herstellverfahren für Verbindungen und Zubereitungen.

Ein Einsatzgebiet der erfindungsgemäßen Verbindungen ist beispielsweise die Kosmetik. Aufgabe pflegender Kosmetik ist es, nach Möglichkeit den Eindruck einer jugendlichen Haut zu erhalten. Prinzipiell stehen verschiedene Wege offen, um diesen Weg zu erreichen. So können bereits vorhandene Schädigungen der Haut, wie unregelmäßige Pigmentierung oder Faltenbildung, durch abdeckende Puder oder Cremes ausgeglichen werden. Ein anderer Ansatzpunkt ist, die Haut vor Umwelteinflüssen zu schützen, die zu einer dauerhaften Schädigung und damit Alterung der Haut führen. Die Idee ist also, vorbeugend einzugreifen und dadurch den Alterungsprozess hinauszuzögern. Ein Beispiel sind hierfür UV-Filter, welche durch Absorption bestimmter Wellenlängenbereiche eine Schädigung der Haut vermeiden oder zumindest vermindern. Während bei UV-Filtern das schädigende Ereignis, die UV-Strahlung, von der Haut abgeschirmt wird, versucht man bei einem weiteren Weg, die natürlichen Abwehr- bzw. Reparaturmechanismen der Haut gegen das schädigende Ereignis zu unterstützen. Schließlich verfolgt man als weiteren Ansatzpunkt die mit zunehmendem Alter sich abschwächenden Abwehrfunktionen der Haut gegen schädigende Einflüsse auszugleichen, indem Substanzen von außen zugeführt werden, die diese nachlassende Abwehr- bzw. Reparaturfunktion ersetzen können. Beispielsweise besitzt die Haut die Fähigkeit, Radikale, die durch äußere oder innere Stressfaktoren erzeugt werden, abzufangen. Diese Fähigkeit schwächt sich mit zunehmendem Alter ab, wodurch sich der Alterungsprozess mit zunehmendem Alter beschleunigt.

Eine weitere Schwierigkeit bei der Herstellung von Kosmetika besteht darin, dass Wirkstoffe, die in kosmetische Zubereitungen eingearbeitet werden sollen, oftmals nicht stabil sind und in der Zubereitung geschädigt werden können. Die Schädigungen können beispielsweise durch eine Reaktion mit Luftsauerstoff oder durch die Absorption von UV-Strahlen verursacht werden. Die so geschädigten Moleküle können durch ihre Strukturänderung z.B. ihre Farbe ändern und/oder ihre Wirksamkeit verlieren. Entsprechende schwierigkeiten tretetn allgemein bei der Herstellung, Lagerung oder Anwendung von Zubereitungen enthaltend oxidationsempfindliche Inhaltsstoffe auf.

Ein bekannter Weg, die beschriebenen Probleme zu behandeln besteht im Zusatz von Antioxidantien zu den Zubereitungen.

Laut CD Römpp Chemie Lexikon - Version 1.0, Stuttgart/New York: Georg Thieme Verlag 1995 handelt es sich bei Antioxidantien um Verbindungen, die unerwünschte, durch Sauerstoff-Einwirkungen u.a. oxidative Prozesse bedingte Veränderungen in den zu schützenden Stoffen hemmen oder verhindern. Einsatzgebiete sind z.B. in Kunststoffen und Kautschuk zum Schutz gegen Alterung; in Fetten zum Schutz vor Ranzigkeit, in Ölen, Viehfutter, Autobenzin und Düsentreibstoffen zum Schutz gegen Verharzung, in Transformatoren- und Turbinenöl gegen Schlammbildung, in Aromastoffen gegen Geruchsverschlechterung. Als Antioxidantien wirksam sind u.a. durch sterisch hindernde Gruppen substituierte Phenole, Hydrochinone, Brenzcatechine, Aromaten, Amine sowie deren Metall-Komplexe. Die Wirkung der Antioxidantien besteht laut Römpp meist darin, daß sie als Radikalfänger für die bei der Autoxidation auftretenden freien Radikale wirken.

Es besteht jedoch weiterhin Bedarf nach hautverträglichen Antioxidantien, die sich auch zum Einsatz in hautpflegenden Zubereitungen eignen.

Aufgabe der Erfindung ist es daher, eine Zusammensetzung zur Verfügung zu stellen, welche eine schützende Wirkung gegen UV-Strahlen aufweist und/oder eine schützende Wirkung gegen oxidativen Stress auf Körperzellen ausübt und/oder einer Alterung der Haut entgegenwirkt.

Ein erster Gegenstand der vorliegenden Erfindung ist daher die Verwendung von Verbindungen der Formel Ia, Ib, Ic oder Id,

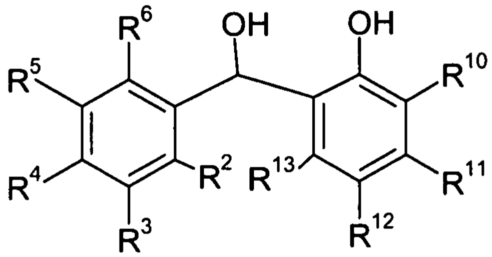

Ia

,

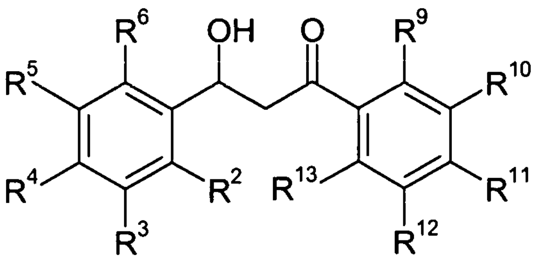

Ib

,

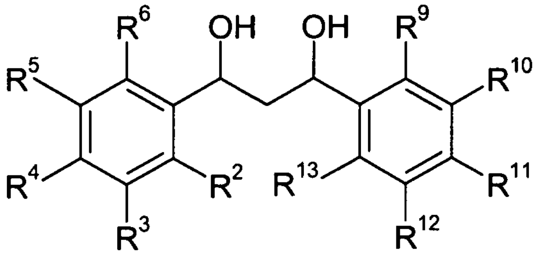

Ic

,

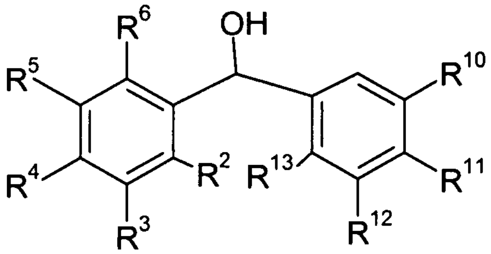

Id

,

wobei

$R^2$ bis $R^6$ und $R^9$ bis $R^{13}$ jeweils unabhängig voneinander ausgewählt sind aus H,
OH,
geradkettigen oder verzweigten $C_1$- bis $C_{20}$-Alkoxygruppen, wobei die Alkylketten jeweils auch durch Sauerstoff oder Stickstoff unterbrochen sein können,
geradkettigen oder verzweigten $C_1$- bis $_{20}$-Alkylgruppen, wobei die Alkylketten jeweils auch durch Sauerstoff oder Stickstoff unterbrochen sein können,
geradkettigen oder verzweigten $C_3$- bis $C_{20}$-Alkenylgruppen,
geradkettigen oder verzweigten $C_1$- bis $C_{20}$-Hydroxyalkylgruppen, wobei die Hydroxygruppe an ein primäres oder sekundäres Kohlenstoffatom der Kette gebunden sein kann und weiter die Alkylketten jeweils auch durch Sauerstoff oder Stickstoff unterbrochen sein können, geradkettigen oder verzweigten $C_1$- bis $C_{20}$-Hydroxyalkoxygruppen, wobei die Hydroxygruppe(n) an primäre oder sekundäre Kohlenstoffatome der Kette gebunden sein können und weiter die Alkylkette auch durch Sauerstoff unterbrochen sein kann,
geradkettigen oder verzweigten $C_1$- bis $C_{20}$-Alkylaminogruppen, geradkettigen oder verzweigten $C_1$- bis $C_{20}$-Dialkylaminogruppen,
oder $R^2$ bis $R^6$ und $R^9$ bis $R^{13}$ stehen jeweils unabhängig voneinander für eine Carbonsäure-, Phosphorsäure-, Sulfonsäure-, Schwefelsäure- oder Sulfonfunktion, die optional mit geradkettigen oder verzweigten $C_1$-bis $_{20}$-Alkylgruppen bzw. geradkettigen oder verzweigten $C_3$- bis $C_{20}$-Alkenylgruppen verestert oder alkyliert sein kann,
oder es sich um Salze der Verbindungen der Formel Ia bis Id handelt, als Antioxidans zur Herstellung kosmetischer oder pharmazeutischer, insbesondere dermatologischer Zubereitungen oder von Nahrungsmitteln bzw. Nahrungsergänzungsmitteln oder zur Herstellung von Haushaltsprodukten.

Als Gegenionen für die Salze können dabei alle für den entsprechenden Einsatzzweck unbedenklichen Anionen eingesetzt werden. Vorteilhaft ist es dabei, wenn es sich um Salze starker Säuren handelt. Insbesondere bevorzugt ist es erfindungsgemäß, wenn es sich bei den Salzen um Chloride oder Bromide handelt.

Die beschriebenen Verbindungen können erfindungsgemäß als Wirkstoff zur topischen Anwendung bzw. zur Herstellung kosmetischer, dermatologischer oder pharmazeutischer Zubereitungen oder zur Herstellung von Haushaltsprodukten verwendet werden. Sie können auch zur Herstellung von Nahrungsmitteln oder Nahrungsergänzungsmitteln verwendet werden. Die beschriebenen Verbindungen können zum Produktschutz eingesetzt werden. Produktschutz bedeutet dabei im Sinne dieser Anmeldung insbesondere den Schutz oxidationsempfindlicher Formulierungsbestandteile wie organische oder anorganische Farbstoffe, Antioxidantien, Vitamine, Parfumkomponenten, Ölkomponenten oder Matrixbestandteile, wie Emulgatoren, Verdicker, Filmbildner und Tenside. Die entsprechende Verwendung ist Gegenstand dieser Anmeldung.

Erfindungsgegenstand ist auch die Verwendung der Verbindungen zur Herstellung kosmetischer oder pharmazeutischer, insbesondere dermatologischer Zubereitungen oder von Nahrungsmitteln bzw. Nahrungsergänzungsmitteln oder zur Herstellung von Haushaltsprodukten.

Vorzugsweise werden dabei Verbindungen der Formeln Ia bis Id eingesetzt, bei denen $R^4$ und $R^{11}$ jeweils unabhängig voneinander ausgewählt sind aus H, geradkettigen oder verzweigten $C_1$-bis $C_{20}$-Alkoxygruppen, geradkettigen oder verzweigten $C_1$- bis $_{20}$-Alkylgruppen oder geradkettigen oder verzweigten $C_1$- bis $C_{20}$-Dialkylaminogruppen.

$R^4$ und $R^{11}$ sind jeweils unabhängig voneinander besonders bevorzugt ausgewählt aus H, geradkettigen oder verzweigten $C_1$- bis $C_4$-Alkoxygruppen, insbesondere Methoxy, Isopropoxy und tert-Butoxy, geradkettigen oder verzweigten $C_1$- bis $C_6$-Alkylgruppen, insbesondere Methyl, Isopropyl und tert-Butyl, und $C_1$- bis $C_4$-Dialkylaminogruppen, insbesondere Dimethylamino oder Diethylamino.

Vorzugsweise werden dabei Verbindungen der Formeln Ia bis Id eingesetzt, bei denen $R^3$, $R^5$, $R^9$, $R^{10}$ und $R^{12}$ H bedeutet.

Eine weitere bevorzugte Gruppe an Verbindungen sind Verbindungen der Formeln Ia bis Id, in denen $R^3$ oder $R^{12}$ jeweils unabhängig voneinander H, $SO_3H$ oder Sulfonat bedeuten. Ganz besonders bevorzugt ist $R^{12}$ $SO_3H$.

Vorzugsweise werden dabei Verbindungen der Formeln Ia bis Id eingesetzt, bei denen $R^2$, $R^6$ und $R^{13}$ H bedeutet.

Weitere Kombinationen sind in den Ansprüchen offenbart. Insbesondere bevorzugt kann in einer Erfindungsvariante die Verwendung mindestens einer Verbindung der Formeln Ia bis Id sein, die, dadurch gekennzeichnet ist, dass
$R^4$ ausgewählt wird aus H, $C_1$- bis $C_4$-Alkoxygruppen oder $C_1$- bis $C_4$-Dialkylaminogruppen,
$R^{11}$ ausgewählt wird aus geradkettigen oder verzweigten $C_1$- bis $C_6$-Alkylgruppen, $C_1$- bis $C_4$-Dialkylaminogruppen oder $C_1$- bis $C_4$-Alkoxygruppen.

Insbesondere bevorzugt kann in einer Erfindungsvariante die Verwendung mindestens einer Verbindung der Formeln Ia bis Id sein, die, dadurch gekennzeichnet ist, dass
$R^6$ $C_1$- bis $C_{10}$-Alkoxycarbonyl bedeutet.

Insbesondere bevorzugt kann in einer Erfindungsvariante die Verwendung mindestens einer Verbindung der Formeln Ia bis Id sein, die, dadurch gekennzeichnet ist, dass mindestens eine Gruppe aus $R^2$, $R^6$ und $R^{13}$ für OH steht. Diese Verbindungen zeigen eine besonders ausgeprägte antioxidative Leistung.

Insbesondere kann in einer weiteren Erfindungsvariante die Verwendung mindestens einer Verbindung der Formeln Ia bis Id bevorzugt sein, die, dadurch gekennzeichnet ist, dass mindestens eine Gruppe aus $R^4$ und $R^{11}$ für t-Butyl steht.

Weiter kann erfindungsgemäß die Verwendung mindestens einer Verbindung der Formeln Ia bis Id mit langkettigen Kohlenwasserstoffresten, insbesondere verzweigten langkettigen Kohlenwasserstoffresten bevorzugt sein. Diese Verbindungen sind oft mit Trägersubstanzen, wie insbesondere Ölen besonders gut mischbar und lassen sich so besonders leicht in Formulierungen einsetzen. Besonders bevorzugt ist es in dieser Erfindungsvariante, wenn mindestens ein Rest aus $R^2$ bis $R^6$ und $R^9$ bis $R^{13}$ steht für einen verzweigten oder unverzweigten $C_{7-30}$-Alkyl-oder $C_{6-30}$-Hydroxyalkylrest oder einen Ester oder Ether enthaltend einen solchen Rest.

In einer weiteren Erfindungsvariante kann es bevorzugt sein, wenn Verbindungen der Formeln Ia bis Id verwendet werden, die dadurch gekennzeichnet sind, dass $R^4$ steht für einen verzweigten oder unverzweigten $C_{1-20}$-Alkoxy oder verzweigten oder unverzweigten $C_{2-20}$-Alkylenoxy-Spacer, der über ein Si-Atom an eine Oligo- oder Polysiloxankette gebunden ist, die ein oder mehrere Verbindungen der Formel I enthält, wobei $R^4$ vorzugsweise steht für einen Propanyloxy-, Isopropanyloxy-, Propenyloxy-, Isopropenyloxy- oder insbesondere einen Allyloxy-Spacer, wobei vorzugsweise ein Silicium-Atom an das 1-C oder an das 2-C der Spacerdoppelbindung gebunden.

Insbesondere bevorzugt ist dabei die Verwendung mindestens einer Verbindung der Formeln Ia bis Id, die ausgewählt ist aus 2-(Hydroxy-phenyl-methyl)-5-diethylamino-phenol, 2-(Hydroxy-phenyl-methyl)-phenol, 2-(Hydroxy-phenyl-methyl)-5-sulfo-phenol, 2-(Hydroxy-phenyl-methyl)-5-methoxy-4-sulfo-phenol, 2-(Hydroxy-phenyl-methyl)-5-methoxy-phenol, 2-[(4-Diethylamino-2-hydroxy-phenyl)-hydroxy-methyl]-benzoesäure-ethylester, 2-[(4-Diethylamino-2-hydroxy-phenyl)-hydroxy-methyl]-benzoesäure-hexylester, 2-[(4-Diethylamino-2-hydroxy-phenyl)-hydroxy-methyl]-benzoesäure-ethylhexylester, 2-[(2-hydroxy-phenyl)-hydroxy-methyl]-benzoesäure-ethylhexylester, 3-Hydroxy-1,3-diphenyl-propan-1-on, 1-(4-tert-butyl-phenyl)-3-hydroxy-3-phenyl-propan-1-on, 1-(4-tert-butyl-phenyl)-3-hydroxy-3-(4-methoxy-phenyl)-propan-1-on, 3-(4-tert-butyl-phenyl)-3-hydroxy-1-phenyl-propan-1-on, 3-(4-tert-butyl-phenyl)-3-hydroxy-1-(4-methoxy-phenyl)-propan-1-on oder 3-(4-methoxy-phenyl)-3-hydroxy-1-(4-methoxy-phenyl)-propan-1-on.

Ein weiterer Gegenstand der vorliegenden Erfindung sind dabei die neuen Verbindungen der Formel Ib oder Ic

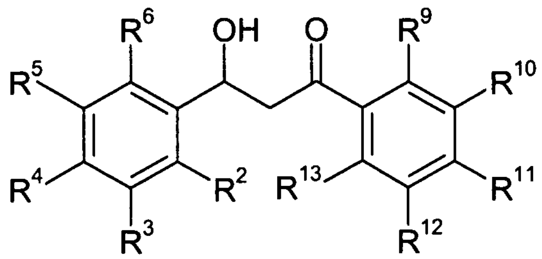

Ib

,

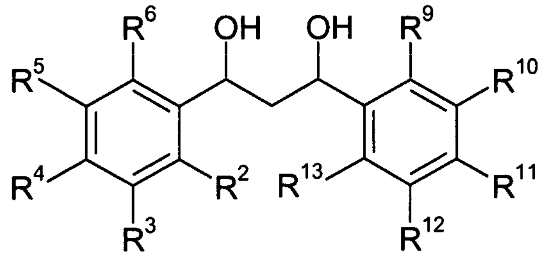

Ic

,

wobei

$R^4$ und $R^{11}$ jeweils unabhängig voneinander H, geradkettige oder verzweigte $C_1$- bis $C_{20}$-Alkylgruppe, geradkettige oder verzweigte $C_1$- bis $C_{20}$-Alkoxygruppe oder geradkettige oder verzweigte $C_1$- bis $C_{20}$-Dialkylaminogruppe,
$R^6$ H oder eine Carbonsäure-, Phosphorsäure-, Sulfonsäure-, Schwefelsäure- oder Sulfonfunktion, die mit geradkettigen oder verzweigten $C_1$- bis $C_{20}$-Alkylgruppen oder geradkettigen oder verzweigten $C_3$- bis $C_{20}$-Alkenylgruppen verestert oder alkyliert sein kann und
$R^2$, $R^3$, $R^5$, $R^9$, $R^{10}$, $R^{12}$ und $R^{13}$ H bedeuten.

Ausgewählte bevorzugte Verbindungen sind die Verbindungen der Formeln (1) bis (6):

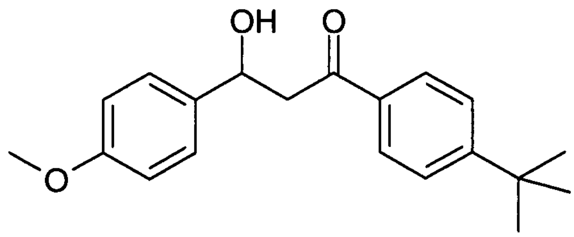

(1)

,

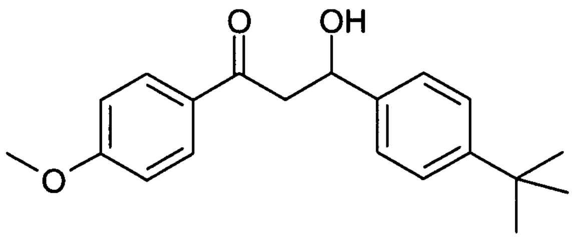

(2)

oder

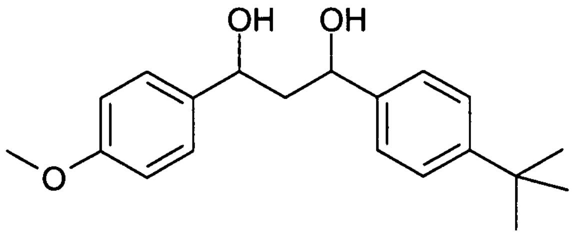

(3)

oder Salze der Verbindungen,
sowie

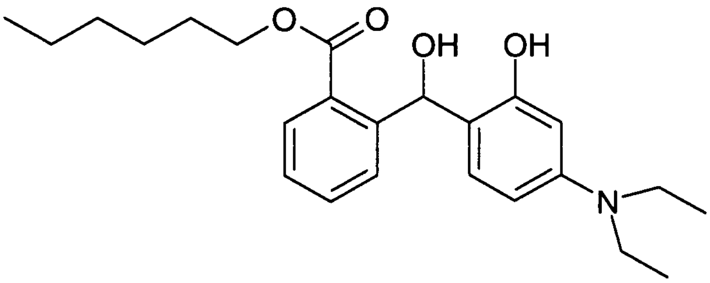

(4)

,

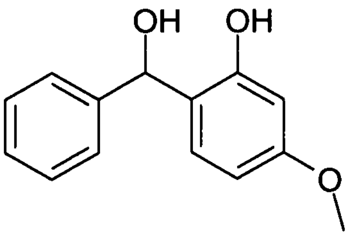

(5)

oder

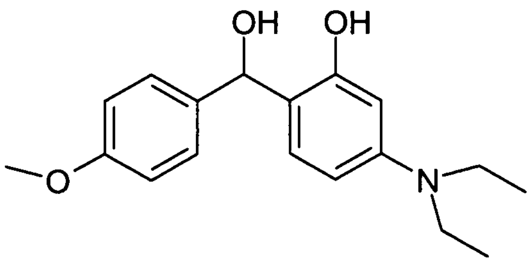

(6)

oder Salze der Verbindungen.

Eine besonders geeignete Gruppe von Verbindungen, die erfindungsgemäß verwendet werden können, sind neben den Verbindungen der Formeln (1) bis (6) auch die Verbindungen der Formeln (7) bis (15)

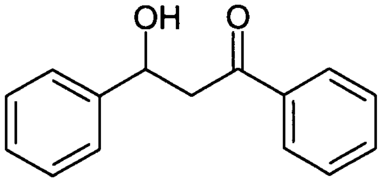

(7)

,

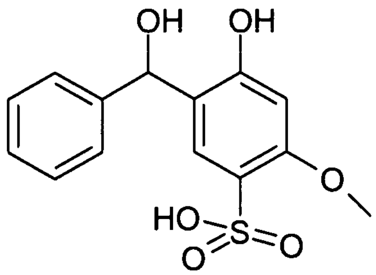

(8)

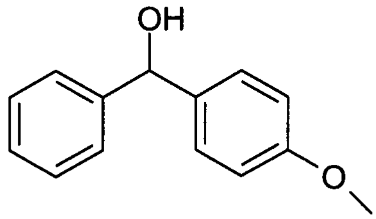

(9)

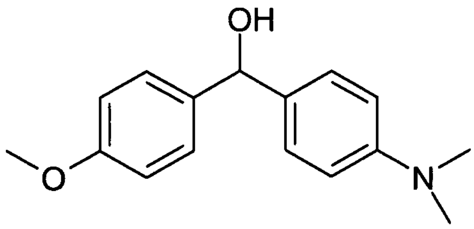

(10)

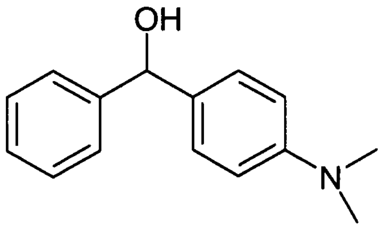

(11)

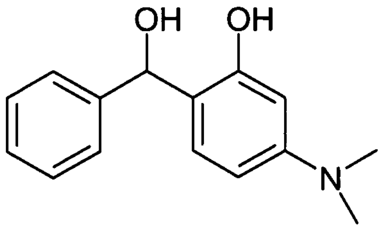

(12)

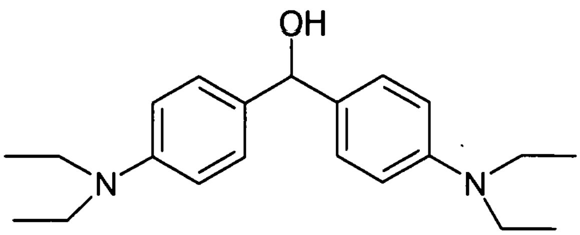

(13)

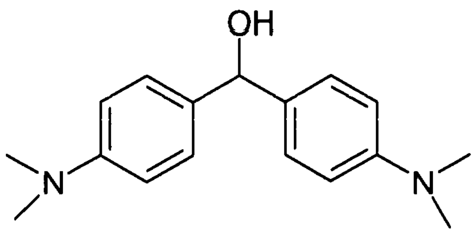

(14)

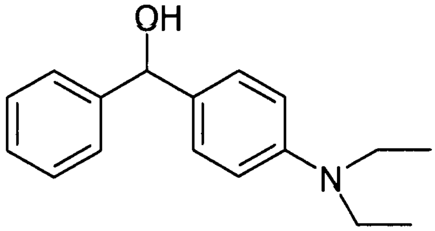

(15)

Ein weiterer Erfindungsgegenstand sind Zubereitungen enthaltend mindestens eine Verbindung der Formeln Ia bis Id.

Bei den Zubereitungen handelt es sich dabei üblicherweise entweder um topisch anwendbare Zubereitungen, beispielsweise kosmetische oder dermatologische Formulierungen, oder um Nahrungsmittel bzw. Nahrungsergänzungsmittel oder um Haushaltsprodukte. Die Zubereitungen enthalten in diesem Fall einen kosmetisch oder dermatologisch, Nahrungsmittel-geeigneten oder Haushaltsproduktegeeigneten Träger und je nach gewünschtem Eigenschaftsprofil optional weitere geeignete Inhaltsstoffe.

Im Sinne der vorliegenden Erfindung wird neben dem Begriff Zubereitung gleichbedeutend auch der Begriff Formulierung verwendet.

Vorteile der erfindungsgemäßen Verbindungen bzw. Verwendung von erfindungsgemäßen Verbindungen bzw. der erfindungsgemäßen Zusammensetzungen können dabei insbesondere sein:

- eine antioxidante Wirkung gegen Radikale, die z.B. durch UV-Licht oder durch thermolytische Prozesse, wie dem Rauchen, induziert werden, wie z.B. gegen das Superoxidradikalanion oder das NO-Radikal, bzw. gegen reaktive Sauerstoffspezies, wie z.B. gegen Singuletsauerstoff und Peroxide,
- bevorzugte Verbindungen vereinigen in sich eine starke antioxidante Aktivität in Kombination mit einer hohen molekularen Stabilität,
- eine produktstabilisierende Wirkung auf kosmetische, pharmazeutische, insbesondere dermatologische Produkte oder Haushaltsprodukte bzw. Nahrungsmittel und Nahrungsergänzungsmittel, insbesondere solche, die Farb-, Konsistenz- oder Geruchsstoffe enthalten,
- bevorzugte Verbindungen der Formel Ia, Ib, Ic oder Id eignen sich als Ölkomponente in Zubereitungen,
- bevorzugte Verbindungen der Formel Ia, Ib, Ic oder Id eignen sich zur Verbesserung von galenischen Eigenschaften, wie beispielsweise des Hautgefühls, von Zubereitungen,
- bevorzugte Verbindungen der Formel Ia, Ib, Ic oder Id zeigen gute Löslichkeits- und Lösungsmitteleigenschaften, vorzugsweise z.B. als Lösungsmittel für kristalline Komponenten,
- eine bevorzugte Gruppe erfindungsgemäßer Verbindungen kann auch einen Hautbräunung hervorrufen bzw. die Wirkung hautbräunender Substanzen, wie Dihydroxyaceton, verbessern,
- die gute Hautverträglichkeit,
- eine produktstabilisierende Wirkung auf Pigmente und Lacke,
- bevorzugte Verbindungen der Formel I eignen sich zur Erzeugung bzw. Erhöhung ("boost"-Effekt) von Lichtschutzfaktoren, wie LSF, SPF, PPD oder IPD, oder Radikalschutzfaktoren,
- eine stabilisierende Wirkung auf autooxidierbare Polyethylenglycol (PEG)- oder Polyglycerin (PG)-Derivate, wie insbesondere PEG- oder PG-haltige Emulgatoren, wie sie weiter unten in dieser Anmeldung genannt werden, bzw. eine Reduktion der schädigenden Wirkung der Abbauprodukte autooxidierbarer Polyethylenglycol (PEG)- oder Polyglycerin (PG)-Derivate,
- eine stabilisierende Wirkung auf Farb-, Konsistenz- oder Geruchsstoffe, oder auf Antioxidantien oder Vitamine, und UV Filter sowie Titandioxid-haltige Pigmente, insbesondere in kosmetische, pharmazeutische, insbesondere dermatologische Produkten oder Haushaltsprodukten bzw. Nahrungsmitteln und Nahrungsergänzungsmitteln,
- während die meisten Antioxidantien nach Reaktion mit Radikalen wirkungslos werden, zeigen bevorzugte Verbindungen der Formel Ia, Ib, Ic oder Id nach dieser Reaktion UV-filternde Wirkung und setzen so ihre Schutzfunktion fort,

- bevorzugte erfindungsgemäße Verbindungen mit antioxidanten Eigenschaften können auch zur Pigmentierungskontrolle eingesetzt werden, da sie eine aufhellende Wirkung auf Hautpartien haben können.

Zusätzlich sind bevorzugte der hier beschriebenen Verbindungen farblos oder nur schwach gefärbt und führen so nicht oder nur in geringer Weise zu Verfärbungen der Zubereitungen.

Wie bereits oben ausgeführt sind Zubereitungen enthaltend mindestens einen für kosmetische oder dermatologische Zubereitungen oder Haushaltsprodukte geeigneten Träger und zumindest eine Verbindung der o. g. Formeln Ia bis Id ein weiterer Gegenstand der vorliegenden Erfindung.

Insbesondere bevorzugt ist es dabei, wenn die Zubereitung mindestens eine Verbindung der Formel Ia en, Ib en oder Id en

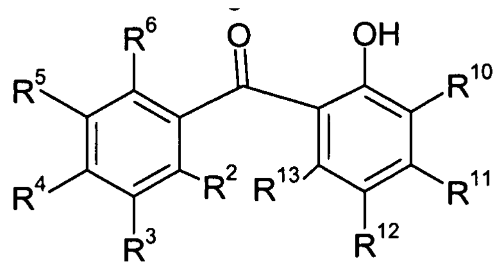

Ia en

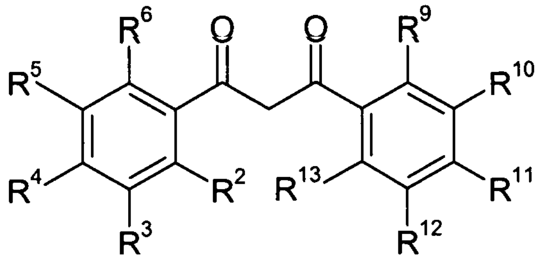

Ib en

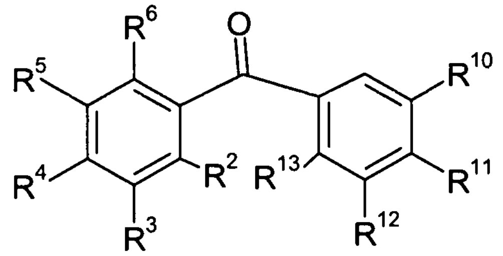

Id en

enthält, wobei die Reste $R^1$-$R^6$ und $R^9$-$R^{13}$ jeweils unabhängig voneinander und unabhängig von den Resten der Verbindungen nach Formel Ia, Ib, Ic oder Id, die oben für die Verbindungen der Formeln Ia, Ib, Ic oder Id angegebene Bedeutung haben.

Dabei ist es insbesondere bevorzugt, wenn die Reste in der mindestens einen Verbindung nach Formeln Ia bis Id und der mindestens einen Verbindung nach Formel Ia en, Ib en oder Id en identisch sind. In diesem Fall kann die Verbindung gleichzeitig als Reservoir für das UV-Absorptions-Potential der Verbindung nach Formel Ia, Ib, Ic oder Id dienen. D.h. der Einsatz der Verbindungen der Formel Ia, Ib, Ic oder Id ermöglicht so eine Reduktion der Einsatzkonzentration des UV Filters nach Formel I en. Die Abstimmung der Einsatzkonzentrationen bereitet dem Fachmann dabei keinerlei Schwierigkeiten.

Die Verbindungen der Formel Ia, Ib, Ic oder Id werden erfindungsgemäß typisch in Mengen von 0,01 bis 20 Gew.-%, vorzugsweise in Mengen von 0,1 Gew.-% bis 10 Gew.-% und insbesondere bevorzugt in Mengen von 1 bis 8 Gew.-% eingesetzt. Dabei bereitet es dem Fachmann keinerlei Schwierigkeiten die Mengen abhängig von der beabsichtigten Wirkung der Zubereitung entsprechend auszuwählen.

Damit die erfindungsgemäßen Verbindungen oder die erfindungsgemäß zu verwendenden Verbindungen der Formeln Ia bis Id in einer Zubereitung, ihre positive Wirkung als Radikalfänger auf die Haut besonders gut entwickeln können, kann es bevorzugt sein die erfindungsgemäßen Verbindungen in tiefere Hautschichten eindringen zu lassen. Dazu stehen mehrere Möglichkeiten zur Verfügung. Zum einen können die erfindungsgemäßen Verbindungen eine

ausreichende Lipophilie aufweisen, um durch die äußere Hautschicht in epidermale Schichten vordringen zu können. Als weitere Möglichkeit können in der Zubereitung auch entsprechende Transportmittel, beispielsweise Liposomen, vorgesehen sein, die einen Transport der erfindungsgemäßen Verbindungen durch die äußeren Hautschichten ermöglichen. Schließlich ist auch ein systemischer Transport der erfindungsgemäßen Verbindungen denkbar. Die Zubereitung wird dann beispielsweise so gestaltet, dass sie für eine orale Gabe geeignet ist.

Allgemein wirken die Substanzen der Formel Ia, Ib, Ic oder Id als Radikalfänger. Solche Radikale werden exogen nicht nur durch Sonnenlicht erzeugt, sondern auch durch die Einwirkung reaktiver Substanzen, wie Ozon, Stickoxiden (z. B. Zigarettenrauch) oder Schwermetallbelastung (z. B. in der Nahrung). Weitere Beispiele sind Anoxie, die den Elektronenfluß stromauf der Cytochromoxidasen blockiert und die Bildung von Superoxidradikalarionen bedingt; Entzündungen, die unter anderem mit der Bildung von Superoxidanionen durch die Membran-NADPH-Oxidase der Leukozyten einhergehen, die jedoch auch mit der Bildung (durch Disproportionierung in Gegenwart von Eisen (II)-ionen) der Hydroxyradikale und anderer reaktiver Spezies, die normalerweise beim Phänomen einer Phagocytose beteiligt sind, einhergehen; sowie Lipidautooxidation die im Allgemeinen durch ein Hydroxylradikal initiiert wird und lipidische Alkoxyradikale und Hydroperoxide liefert.

Aufgrund dieser Eigenschaften eignen sich die erfindungsgemäßen Verbindungen bzw. die erfindungsgemäß zu verwendenden Verbindungen der Formeln Ia bis Id in einer Zubereitung allgemein auch zur Immunprotektion und zum Schutz der DNA und RNA. Insbesondere eignen sich die Verbindungen oder die erfindungsgemäß zu verwendenden Verbindungen der Formeln Ia bis Id in einer Zubereitung dabei zum Schutz von DNA und RNA vor oxidativen Angriffen, vor Radikalen und vor Schädigung durch Strahlung, insbesondere UV-Strahlung. Ein weiterer Vorteil der erfindungsgemäßen Verbindungen bzw. erfindungsgemäß zu verwendenden Verbindungen der Formeln Ia bis Id in einer Zubereitung ist der Zellschutz, insbesondere der Schutz von Langerhans-Zellen vor Schäden durch die oben genannten Einflüsse. Alle diese Verwendungen bzw. die Verwendung der erfindungsgemäßen Verbindungen zur Herstellung entsprechend einsetzbarer Zubereitungen sind ausdrücklich auch Gegenstand der vorliegenden Erfindung.

Insbesondere eignen sich bevorzugte Verbindungen der Formeln Ia bis Id bzw. Zusammensetzungen diese Verbindungen enthaltend auch zur Behandlung von Hautkrankheiten, die mit einer Störung der Keratinisierung verbunden sind, die die Differenzierung und Zellprolif-eration betrifft, insbesondere zur Behandlung der Akne vulgaris, Akne comedonicä, der polymorphen Akne, der Akne rosaceae, der nodulären Akne, der Akne conglobata, der alters-bedingten Aknen, der als Neben-wirkung auftretenden Aknen, wie der Akne solaris, der medikamenten-bedingten Akne oder der Akne professionalis, zur Behandlung anderer Störungen der Keratinisierung, insbesondere der Ichtyosen, der ichtyosiformen Zustände, der Darrier-Krankheit, der Keratosis palmoplantaris, der Leukoplasien, der leukoplasiformen Zustände, der Haut- und Schleimhaut-flechten (Buccal) (Lichen), zur Behandlung anderer Hauterkrankungen, die mit einer Störung der Keratinisierung zusammenhängen und eine entzünd-liche und/oder immunoallergische Komponente haben und insbesondere aller Formen der Psoriasis, die die Haut, die Schleimhäute und die Finger und Zehennägel betreffen, und des psoriatischen Rheumas und der Hautatopien, wie Ekzemen oder der respiratorischen Atopie oder auch der Hypertrophie des Zahnfleisches, wobei die Verbindungen ferner bei einigen Entzündungen verwendet werden können, die nicht mit einer Störung der Keratinisierung zusammenhängen, zur Behandlung aller gutartigen oder bösartigen Wucherungen der Dermis oder Epidermis, die gegebenenfalls viralen Ursprungs sind, wie Verruca vulgaris. Veruca plana, Epidermodysplasia verruciformis, orale Papillomatose, Papillomatosis florida, und der Wucherungen, die durch UV-Strahlung hervorgerufen werden können, insbesondere des Epithelioma baso-cellulare und Epithelioma spinocellulare, zur Behandlung anderer Hautkrankheiten, wie der Dermatitis bullosa und der das Kollagen betreffenden Krankheiten, zur Behandlung bestimmter Augenkrankheiten, insbesondere der Hornhauterkrankungen, zur Behebung oder Bekämpfung der lichtbedingten und der mit dem Älterwerden zusammenhängenden Hautalterung, zur Verminderung der Pigmentierungen und der Keratosis actinica und zur Behandlung aller Krankheiten, die mit der normalen Alterung oder der licht-bedingten Alterung zusammenhängen, zur Vorbeugung vor oder der Heilung von Wunden/Narben der Atrophien der Epidermis und/oder Dermis, die durch lokal oder systemisch angewendete Corticosteroide hervorgerufen werden und aller sonstigen Arten der Hautatrophie, zur Vorbeugung vor oder Behandlung von Störungen der Wundheilung, zur Vermeidung oder Behebung von Schwangerschaftsstreifen oder auch zur Förderung der Wundheilung, zur Bekämpfung von Störungen der Talg-produktion, wie Hypersebhorrhö bei Akne oder der einfachen Seborrhö, zur Bekämpfung von oder Vorbeugung von krebsartigen Zuständen oder vor präkanzerogenen Zuständen, insbesondere der promyelozytären Leukämien, zur Behandlung von Entzündungserkrankungen, wie Arthritis, zur Behandlung aller virusbedingten Erkrankungen der Haut oder anderer Bereiche des Körpers, zur Vorbeugung vor oder Behandlung der Alopecie, zur Behandlung von Hautkrankheiten oder Krankheiten anderer Körperbereiche mit einer immuno-logischen Komponente, zur Behandlung von Herz-/Kreislauf-Erkrankungen, wie Arteriosklerose oder Bluthochdruck, sowie des Insulin-unabhängigen Diabetes, zur Behandlung von Hautproblemen, die durch UV-Strahlung hervorgerufen werden.

Die Antioxidanten Wirkungen der Verbindungen der Formeln Ia bis Id können beispielsweise mit dem 2,2-Diphenyl-1-picrylhydrazyl(DPPH)-Assay gezeigt werden. 2,2-Diphenyl-1-picrylhydrazyl ist ein in Lösung stabiles freies Radikal. Das ungepaarte Elektron führt zu einer starken Absorptionsbande bei 515 nm, die Lösung ist dunkel-violett gefärbt. In Gegenwart eines Radikalfängers wird das Elektron gepaart, die Absorption verschwindet und die Entfärbung

verläuft stöchiometrisch unter Berücksichtigung der aufgenommenen Elektronen. Gemessen wird die Extinktion im Photometer. Die antiradikalische Eigenschaft der zu testenden Substanz wird bestimmt, indem man die Konzentration ermittelt, bei der 50 % des eingesetzten 2,2-Diphenyl-1-picrylhydrazyls mit dem Radikalfänger reagiert haben. Ausgedrückt wird diese Konzentration als $EC_{50}$, ein Wert, der unter den gegebenen Messbedingungen als Substanzeigenschaft zu betrachten ist. Verglichen wird die untersuchte Substanz mit einem Standard (z.B. Tocopherol). Der $EC_{50}$-Wert ist dabei ein Maß für die Kapazität der jeweiligen Verbindung Radikale zu fangen. Je niedriger der $EC_{50}$-Wert ist, desto höher ist die Kapazität Radikale zu fangen. Im Sinne dieser Erfindung wird von einer großen oder hohen Kapazität Radikale zu fangen gesprochen, wenn der $EC_{50}$-Wert niedriger als der von Tocopherol.

Ein weiterer wichtiger Aspekt für die Wirkung der Antioxidantien ist die Zeit in der dieser $EC_{50}$-Wert erreicht wird. Diese Zeit gemessen in Minuten ergibt den $T_{EC50}$-Wert, der eine Aussage über die Geschwindigkeit zulässt, mit der diese Antioxidantien Radikale fangen. Im Sinne dieser Erfindungen gelten Antioxidantien, die diesen Wert innerhalb von weniger als 60 Minuten erreichen als schnell, solche die den $EC_{50}$-Wert erst nach mehr als 120 Minuten erreichen als zeitverzögert wirkend.

Die antiradikalische Effizienz (AE) (beschrieben bei C. Sanchez-Moreno, J.A. Larrauri und F. Saura-Calixto in J. Sci. Food Agric. 1998, 76(2), 270-276.) ergibt sich aus den oben genannten Größen nach folgender Beziehung:

$$AE = \frac{1}{EC_{50} T_{EC50}}$$

Eine niedrige AE (x$10^3$) liegt im Bereich bis etwa 10, von einer mittleren AE wird im Bereich von 10 bis 20 gesprochen und eine hohe AE liegt erfindungsgemäß bei Werten oberhalb 20 vor.

Dabei kann es insbesondere bevorzugt sein, schnell wirkende Antioxidantien mit solchen mit langsamer oder zeitverzögerter Wirkung zu kombinieren. Dabei sind typische Gewichtsverhältnisse der schnell wirkenden Antioxidantien zu zeitverzögert wirkenden Antioxidantien im Bereich 10:1 bis 1:10, vorzugsweise im Bereich 10:1 bis 1:1 und für hautschützende Zubereitungen insbesondere bevorzugt im Bereich 5:1 bis 2:1. In anderen ebenfalls bevorzugten Zubereitungen kann es im Sinne einer Wirkungsoptimierung allerdings von Vorteil sein, mehr zeitverzögert wirkende Antioxidantien als schnell wirkende Antioxidanten vorliegen. Typische Zusammensetzungen zeigen dann Gewichtsverhältnisse der schnell wirkenden Antioxidantien zu zeitverzögert wirkenden Antioxidantien im Bereich 1:1 bis 1:10, vorzugsweise im Bereich 1:2 bis 1:8.

Die schützende Wirkung gegen oxidativen Stress bzw. gegen die Einwirkung von Radikalen kann also weiter verbessert werden, wenn die Zubereitungen ein oder mehrere weitere Antioxidantien enthalten, wobei es dem Fachmann keinerlei Schwierigkeiten bereitet geeignet schnell oder zeitverzögert wirkende Antioxidantien auszuwählen.

In einer bevorzugten Ausführungsform der vorliegenden Erfindungen handelt es sich bei der Zubereitung daher um eine Zubereitung zum Schutz von Körperzellen gegen oxidativen Stress, insbesondere zur Verringerung der Hautalterung, dadurch gekennzeichnet, dass sie neben den ein oder mehreren Verbindungen nach den Formeln Ia bis Id, vorzugsweise ein oder mehrere weitere Antioxidantien enthält.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. $\alpha$-Carotin, $\beta$-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, $\gamma$-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis $\mu$mol/kg), ferner (Metall-) Chelatoren, (z.B. $\alpha$-Hydroxyfettsäuren, Palmitinsäure, Phytinsäure, Lactoferrin), $\alpha$-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, $\alpha$-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, $ZnSO_4$), Selen und dessen Derivate (z.B. Selenmethionin), Stilbene und deren Derivate (z.B. Stilbenoxid, trans-Stilbenoxid).

Geeignete Antioxidantien sind auch in der WO 2006/111233 und WO 2006/111234 beschrieben.

Geeignete Antioxidantien sind auch Verbindungen der allgemeinen Formeln A oder B

A 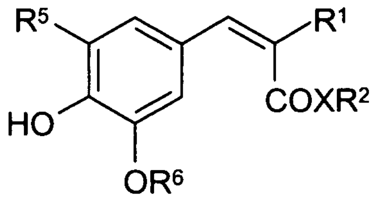 oder 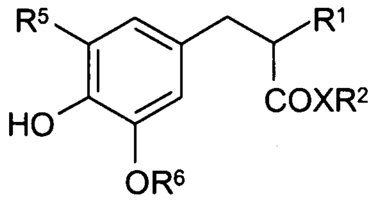 B ,

worin

$R^1$ aus der Gruppe $-C(O)CH_3$, $-CO_2R^3$, $-C(O)NH_2$ und $-C(O)N(R^4)_2$ ausgewählt werden kann,
X O oder NH,
$R^2$ lineares oder verzweigtes Alkyl mit 1 bis 30 C-Atomen,
$R^3$ lineares oder verzweigtes Alkyl mit 1 bis 20 C-Atomen,
$R^4$ jeweils ungabhängig voneinander H oder lineares oder verzweigtes Alkyl mit 1 bis 8 C-Atomen,
$R^5$ lineares oder verzweigtes Alkyl mit 1 bis 8 C-Atomen oder lineares oder verzweigtes Alkoxy mit 1 bis 8 C-Atomen und
$R^6$ lineares oder verzweigtes Alkyl mit 1 bis 8 C-Atomen bedeutet, vorzugsweise Derivate der 2-(4-Hydroxy-3,5-dimethoxybenzyliden)-malonsäure und/oder 2-(4-Hydroxy-3,5-dimethoxybenzyl)-malonsäure, besonders bevorzugt 2-(4-Hydroxy-3,5-dimethoxybenzyliden)-malonsäure-bis-(2-ethylhexyl)ester (z.B. Oxynex® ST Liquid) und/oder 2-(4-Hydroxy-3,5-dimethoxybenzy)-malonsäure-bis-(2-ethylhexyl)ester (z.B. RonaCare® AP).

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure (z.B. (z.B. Oxynex® AP), natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L-(+)-Ascorbylpalmitat, Zitronensäure und Lecithin (z.B. Oxynex® LM) oder Butylhydroxytoluol (BHT), L-(+)-Ascorbylpalmitat und Zitronensäure (z.B. Oxynex® 2004). Derartige Antioxidantien werden mit erfindungsgemäßen Verbindungen in solchen Zusammensetzungen überlicherweise in Verhältnissen im Bereich von 1000:1 bis 1:1000, bevorzugt in Mengen von 100:1 bis 1:100 eingesetzt.

Die Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin $B_1$), Riboflavin (Vitamin $B_2$), Nicotinsäureamid, Vitamin C (Ascorbinsäure), Vitamin D, Ergocalciferol (Vitamin $D_2$), Vitamin E, DL-α-Tocopherol, Tocopherol-E-Acetat, Tocopherolhydrogensuccinat, Vitamin $K_1$, Esculin (Vitamin P-Wirkstoff), Thiamin (Vitamin $B_1$), Nicotinsäure (Niacin), Pyridoxin, Pyridoxal, Pyridoxamin, (Vitamin $B_6$), Panthothensäure, Biotin, Folsäure und Cobalamin (Vitamin $B_{12}$) in den erfindungsgemäßen kosmetischen Zubereitungen enthalten, insbesondere bevorzugt Vitamin-A-Palmitat, Retinol, Vitamin C und dessen Derivaten, DL-α-Tocopherol, Tocopherol-E-Acetat, Nicotinsäure, Pantothensäure und Biotin. Vitamine werden dabei mit erfindungsgemäßen Verbindungen überlicherweise in Verhältnissen im Bereich von 1000:1 bis 1:1000, bevorzugt in Mengen von 100:1 bis 1:100 eingesetzt.

Dabei hat sich gezeigt, dass Antioxidantien, wie beispielsweise beta-Carotin und Tocopherol die Konversion der erfindungsgemäßen Verbindungen der Formeln Ia bis Id zu UV-filternden Verbindungen beschleunigen können. Daher ist ein weiterer Gegenstand der vorliegenden Anmeldung die Verwendung von Antioxidantien zur Aktivierung der erfindungsgemäßen Verbindungen. Erfindungsgemäß bevorzugt einzusetzende Verbindungen weisen - nach Bestrahlung - eine UV-Absorption im UV-A- und oder UV-B-Bereich auf. Unter den erfindungsgemäß einzusetzenden Verbindungen finden sich dabei Vorstufen von Breitband-UV-Filtern, die alleine oder in Kombination mit weiteren UV-Filtern eingesetzt werden können. Andere ebenfalls bevorzugte erfindungsgemäße Verbindungen sind Vorstufen von UV-Filtern mit einem Absorptionsmaximum im Grenzbereich zwischen der UV-B- und der UV-A-Strahlung. Als UV-A-II-Filter können sie daher vorteilhaft das Absorptionsspektrum von handelsüblichen UV-B- bzw. UV-A-I-Filtern ergänzen.

Weiter haben bevorzugte Verbindungen Vorteile bei der Einarbeitung in die Zubereitungen:

- geradkettige oder verzweigte $C_1$- bis $C_{20}$-Alkoxygruppen, insbesondere die langkettigen Alkoxyfunktionen, wie Ethylhexyloxy-Gruppen erhöhen die Öllöslichkeit der Verbindungen.
- zum Teil liegen solche Verbindungen als Ölkomponenten vor und können einfach in die Zubereitung eingearbeitet werden bzw. können für andere Rezepturbestandteile als Lösungsmittel fungieren.

Die Zubereitungen können in ebenfalls bevorzugten Ausführungsformen jedoch auch in der Zubereitungs-Matrix schlecht oder nicht lösliche Verbindungen der Formeln Ia bis Id enthalten. In diesem Fall liegen die Verbindungen vorzugsweise in feinteiliger Form in der kosmetischen Zubereitung dispergiert vor.

Insbesondere bevorzugte Zubereitungen können auch als Sonnenschutzmittel dienen und enthalten dann neben den erfindungsgemäßen Verbindungen auch UV-Filter.

Bei Einsatz der insbesondere als UV-A-Filter bevorzugten, aber auch als UVB-Filter verwendeten Dibenzoylmethanderivate, oder der insbesondere als UVB-Filter eingesetzten Zimtsäurederivate in Kombination mit den erfindungsgemäßen Verbindungen ergibt sich ein zusätzlicher Vorteil: Die UV-empfindlichen Dibenzoylmethanderivate und Zimtsäurederivate werden durch die Anwesenheit der erfindungsgemäßen Verbindungen zusätzlich stabilisiert. Ein weiterer Gegenstand der vorliegenden Erfindung ist daher die Verwendung der Verbindungen der Formeln Ia bis Id zur Stabilisierung von Dibenzoylmethanderivaten und/oder Zimtsäurederivaten in Zubereitungen.

Prinzipiell kommen alle UV-Filter für eine Kombination mit den Verbindungen der Formel Ia bis Id in Frage. Besonders bevorzugt sind solche UV-Filter, deren physiologische Unbedenklichkeit bereits nachgewiesen ist. Sowohl für UVA wie auch UVB-Filter gibt es viele aus der Fachliteratur bekannte und bewährte Substanzen, z.B.

Benzylidenkampferderivate wie 3-(4'-Methylbenzyliden)-dl-kampfer (z.B. Eusolex® 6300), 3-Benzylidenkampfer (z.B. Mexoryl® SD), Polymere von N-{(2 und 4)-[(2-oxoborn-3-yliden)methyl]benzyl}-acrylamid (z.B. Mexoryl® SW), N,N,N-Trimethyl-4-(2-oxoborn-3-ylidenmethyl)anilinium methylsulfat (z.B. Mexoryl® SK) oder (2-Oxoborn-3-yliden)toluol-4-sulfonsäure (z.B. Mexoryl® SL),
Benzoyl- oder Dibenzoylmethane wie 1-(4-tert-Butylphenyl)-3-(4-methoxyphenyl)propan-1,3-dion (z.B. Eusolex® 9020) oder 4-Isopropyldibenzoylmethan (z.B. Eusolex® 8020),
Benzophenone wie 2-Hydroxy-4-methoxybenzophenon (z.B. Eusolex® 4360) oder 2-Hydroxy-4-methoxybenzophenon-5-sulfonsäure und ihr Natriumsalz (z.B. Uvinul® MS-40),
Methoxyzimtsäureester wie Methoxyzimtsäureoctylester (z.B. Eusolex® 2292), 4-Methoxyzimtsäureisopentylester, z.B. als Gemisch der Isomere (z.B. Neo Heliopan® E 1000),
Salicylatderivate wie 2-Ethylhexylsalicylat (z.B. Eusolex® OS), 4-Isopropylbenzylsalicylat (z.B. Megasol®) oder 3,3,5-Trimethylcyclohexylsalicylat (z.B. Eusolex® HMS),
4-Aminobenzoesäure und Derivate wie 4-Aminobenzoesäure, 4-(Dimethylamino)benzoesäure-2-ethylhexylester (z.B. Eusolex® 6007), ethoxylierter 4-Aminobenzoesäureethylester (z.B. Uvinul® P25),
Phenylbenzimidazolsulfonsäuren, wie 2-Phenylbenzimidazol-5-sulfonsäure sowie ihre Kalium-, Natrium- und Triethanolaminsalze (z.B. Eusolex® 232), 2,2-(1,4-Phenylen)-bisbenzimidazol-4,6-disulfonsäure bzw. deren Salze (z.B. Neoheliopan® AP) oder 2,2-(1,4-Phenylen)-bisbenzimidazol-6-sulfonsäure;
und weitere Substanzen wie

- 2-Cyano-3,3-diphenylacrylsäure-2-ethylhexylester (z.B. Eusolex® OCR),
- 3,3'-(1,4-Phenylendimethylen)-bis-(7,7-dimethyl-2-oxobicyclo-[2.2.1]hept-1-ylmethansulfonsäure sowie ihre Salze (z.B. Mexoryl® SX) und
- 2,4,6-Trianilino-(p-carbo-2'-ethylhexyl-1'-oxi)-1,3,5-triazin (z.B. Uvinul® T 150)
- 2-(4-Diethylamino-2-hydroxy-benzoyl)-benzoesäure hexylester (z.B. Uvinul®UVA Plus, Fa. BASF).

Die in der Liste aufgeführten Verbindungen sind nur als Beispiele aufzufassen. Selbstverständlich können auch andere UV-Filter verwendet werden.

Diese organischen UV-Filter werden in der Regel in einer Menge von 0,5 bis 10 Gewichtsprozent, vorzugsweise 1 - 8 Gew.-%, in kosmetische Formulierungen eingearbeitet.

Weitere geeignete organische UV-Filter sind z.B.

- 2-(2H-Benzotriazol-2-yl)-4-methyl-6-(2-methyl-3-(1,3,3,3-tetramethyl-1-(trimethylsilyloxy)disiloxanyl)propyl)phenol (z.B. Silatrizole®),
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb® HEB),
- $\alpha$-(Trimethylsilyl)-$\omega$-[trimethylsilyl)oxy]poly[oxy(dimethyl [und ca. 6% methyl[2-[p-[2,2-bis(ethoxycarbonyl]vinyl]phenoxy]-1-methylenethyl] und ca. 1,5 % methyl[3-[p-[2,2-bis(ethoxycarbonyl)vinyl)phenoxy)-propenyl) und 0,1 bis 0,4% (methylhydrogen]silylen]] ($n \approx 60$) (CAS-Nr. 207 574-74-1)
- 2,2'-Methylen-bis-(6-(2H-benzotriazol-2-yl)-4-(1,1,3,3-tetramethylbutyl)phenol) (CAS-Nr. 103 597-45-1)
- 2,2'-(1,4-Phenylen)bis-(1H-benzimidazol-4,6-disulfonsäure, Mononatriumsalz) (CAS-Nr. 180 898-37-7) und
- 2,4-bis-{[4-(2-Ethyl-hexyloxy)-2-hydroxyl]-phenyl}-6-(4-methoxyphenyl)-1,3,5-triazin (CAS-Nr. 103 597-45-, 187 393-00-6).

- 4,4'-[(6-[4-((1,1-Dimethylethyl)aminocarbonyl)phenylamino]-1,3,5-triazin-2,4-diyl)dümino]bis(benzoesäure-2-ethylhexylester) (z.B. Uvasorb® HEB),

Weitere geeignete UV-Filter sind auch Methoxyflavone ensprechend der älteren Deutschen Patentanmeldung DE-A-10232595.

Organische UV-Filter werden in der Regel in einer Menge von 0,5 bis 20 Gewichtsprozent, vorzugsweise 1 - 15 Gew.-%, in kosmetische Formulierungen eingearbeitet.

Als anorganische UV-Filter sind solche aus der Gruppe der Titandioxide wie z.B. gecoatetes Titandioxid (z.B. Eusolex® T-2000, Eusolex®T-AQUA; Eusolex® T-AVO), Zinkoxide (z.B. Sachtotec®), Eisenoxide oder auch Ceroxide denkbar. Diese anorganischen UV-Filter werden in der Regel in einer Menge von 0,5 bis 20 Gewichtsprozent, vorzugsweise 2 - 10 Gew.-%, in kosmetische Zubereitungen eingearbeitet.

Bevorzugte Verbindungen mit UV-filternden Eigenschaften sind 3-(4'-Methylbenzyliden)-dl-kampfer, 1-(4-tert-Butylphenyl)-3-(4-methoxy-phenyl)-pro-pan-1,3-dion, 4-Isopropyldibenzoylmethan, 2-Hydroxy-4-meth-oxy-ben-zo--phenon, Methoxyzimtsäureoctylester, 3,3,5-Trimethyl-cyclo-hexylsali-cylat, 4-(Dimethylamino)benzoesäure-2-ethyl-hexylester, 2-Cyano-3,3-di-phenyl-acrylsäure-2-ethylhexylester, 2-Phenyl-benzimidazol-5-sulfonsäure sowie ihre Kalium-, Natrium- und Triethanol-aminsalze.

Durch Kombination von einer oder mehrerer der Verbindungen der Formeln Ia bis Id mit weiteren UV-Filtern kann die Schutzwirkung gegen schädliche Einwirkungen der UV-Strahlung optimiert werden.

Optimierte Zusammensetzungen können beispielsweise die Kombination der organischen UV-Filter 4'-Methoxy-6-hydroxyflavon mit 1-(4-tert-Butylphenyl)-3-(4-methoxyphenyl)propan-1,3-dion und 3-(4'-Methylbenzyliden)-dl-kampfer enthalten. Mit dieser Kombination ergibt sich ein Breitbandschutz, der durch Zusatz von anorganischen UV-Filtern, wie Titandioxid-Mikropartikeln noch ergänzt werden kann.

Alle genannten UV-Filter und die Verbindungen der Formeln Ia bis Id können auch in verkapselter Form eingesetzt werden. Insbesondere ist es von Vorteil organische UV-Filter in verkapselter Form einzusetzen. Im Einzelnen ergeben sich die folgende Vorteile:

- Die Hydrophilie der Kapselwand kann unabhängig von der Löslichkeit des UV-Filters bzw. der Verbindung der Formel I eingestellt werden. So können beispielsweise auch hydrophobe UV-Filter bzw. erfindungsgemäßen Verbindungen in rein wässrige Zubereitungen eingearbeitet werden. Zudem wird der häufig als unangenehm empfundene ölige Eindruck beim Auftragen der hydrophobe UV-Filter enthaltenden Zubereitung unterbunden.
- Bestimmte UV-Filter, insbesondere Dibenzoylmethanderivate, zeigen in kosmetischen Zubereitungen nur eine verminderte Photostabilität. Durch Verkapselung dieser Filter oder von Verbindungen, welche die Photostabilität dieser Filter beeinträchtigen, wie beispielsweise Zimtsäurederivate, kann die Photostabilität der gesamten Zubereitung erhöht werden.
- In der Literatur wird immer wieder die Hautpenetration durch organische UV-Filter und das damit verbundene Reizpotential beim direkten Auftragen auf die menschliche Haut diskutiert. Durch die hier vorgeschlagene Verkapselung der entsprechenden Substanzen wird dieser Effekt unterbunden.
- Allgemein können durch Verkapselung einzelner UV-Filter bzw. erfindungsgemäßen Verbindungen oder anderer Inhaltstoffe Zubereitungsprobleme, die durch Wechselwirkung einzelner Zubereitungsbestandteile untereinander entstehen, wie Kristallisationsvorgänge, Ausfällungen und Agglomeratbildung vermieden werden, da die Wechselwirkung unterbunden wird.

Daher ist es bevorzugt, wenn ein oder mehrere der oben genannten UV-Filter bzw. Verbindungen der Formeln Ia bis Id, in verkapselter Form vorliegen. Vorteilhaft ist es dabei, wenn die Kapseln so klein sind, dass sie mit dem bloßen Auge nicht beobachtet werden können. Zur Erzielung der o.g. Effekte ist es weiterhin erforderlich, dass die Kapseln hinreichend stabil sind und den verkapselten Wirkstoff (UV-Filter) nicht oder nur in geringem Umfang an die Umgebung abgeben.

Geeignete Kapseln können Wände aus anorganischen oder organischen Polymeren aufweisen. Beispielsweise wird in US 6,242,099 B1 die Herstellung geeigneter Kapseln mit Wänden aus Chitin, Chitin-Derivaten oder polyhydroxylierten Polyaminen beschrieben. Erfindungsgemäß besonders bevorzugt einzusetzende Kapseln weisen Wände auf, die durch einen SolGel-Prozeß, wie er in den Anmeldungen WO 00/09652, WO 00/72806 und WO 00/71084 beschrieben ist, erhalten werden können. Bevorzugt sind hier wiederum Kapseln, deren Wände aus Kieselgel (Silica; undefiniertes Silicium-oxid-hydroxid) aufgebaut sind. Die Herstellung entsprechender Kapseln ist dem Fachmann beispielsweise aus den zitierten Patentanmeldungen bekannt, deren Inhalt ausdrücklich auch zum Gegenstand der vorliegenden Anmeldung gehört.

Dabei sind die Kapseln in erfindungsgemäßen Zubereitungen vorzugsweise in solchen Mengen enthalten, die gewährleisten, dass die verkapselten UV-Filter in den oben angegebenen Mengen in der Zubereitung vorliegen.

Die erfindungsgemäßen Zubereitungen enthaltend mindestens eine Verbindung der Formeln Ia bis Id, können darüber hinaus weitere übliche hautschonende oder hautpflegende Wirkstoffe enthalten. Dies können prinzipiell alle den Fachmann bekannten Wirkstoffe sein.

Besonders bevorzugte Wirkstoffe sind Pyrimidincarbonsäuren und/oder Aryloxime.

Pyrimidincarbonsäuren kommen in halophilen Mikroorganismen vor und spielen bei der Osmoregulation dieser Organismen eine Rolle (E. A. Galinski et al., Eur. J. Biochem., 149 (1985) Seite 135-139). Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure und deren Derivate zu nennen. Diese Verbindungen stabilisieren Enzyme und andere Biomoleküle in wässrigen Lösungen und organischen Lösungsmitteln. Weiter stabilisieren sie insbesondere Enzyme gegen denaturierende Bedingungen, wie Salze, extreme pH-Werte, Tenside, Harnstoff, Guanidiniumchlorid und andere Verbindungen.

Ectoin und Ectoin-Derivate wie Hydroxyectoin können vorteilhaft in Arzneimitteln verwendet werden. Insbesondere kann Hydroxyectoin zur Herstellung eines Arzneimittels zur Behandlung von Hauterkrankungen eingesetzt werden. Andere Einsatzgebiete des Hydroxyectoins und anderer Ectoin-Derivate liegen typischerweise in Gebieten in denen z.B. Trehalose als Zusatzstoff verwendet wird. So können Ectoin-Derivate, wie Hydroxyectoin, als Schutzstoff in getrockneten Hefe- und Bakterienzellen Verwendung finden. Auch pharmazeutische Produkte wie nicht glykosylierte, pharmazeutische wirksame Peptide und Proteine z.B. t-PA können mit Ectoin oder seinen Derivaten geschützt werden.

Unter den kosmetischen Anwendungen ist insbesondere die Verwendung von Ectoin und Ectoin-Derivaten zur Pflege von gealterter, trockener oder gereizter Haut zu nennen. So wird in der europäischen Patentanmeldung EP-A-0 671 161 insbesondere beschrieben, dass Ectoin und Hydroxyectoin in kosmetischen Zubereitungen wie Pudern, Seifen, tensidhaltigen Reinigungsprodukten, Lippenstiften, Rouge, Make-Ups, Pflegecremes und Sonnenschutzpräparaten eingesetzt werden.

Dabei wird vorzugsweise eine Pyrimidincarbonsäure gemäß der unten stehenden Formel eingesetzt,

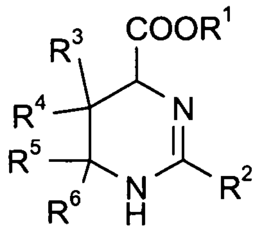

worin $R^1$ ein Rest H oder C1-8-Alkyl, $R^2$ ein Rest H oder C1-4-Alkyl und $R^3$, $R^4$, $R^5$ sowie $R^6$ jeweils unabhängig voneinander ein Rest aus der Gruppe H, OH, $NH_2$ und C1-4-Alkyl sind. Bevorzugt werden Pyrimidincarbonsäuren eingesetzt, bei denen $R^2$ eine Methyl- oder eine Ethylgruppe ist und $R^1$ bzw. $R^5$ und $R^6$ H sind. Insbesondere bevorzugt werden die Pyrimidincarbonsäuren Ectoin ((S)-1,4,5,6-Tetrahydro-2-methyl-4-pyrimidin-carbonsäure) und Hydroxyectoin ((S, S)-1,4,5,6-Tetrahydro-5-hydroxy-2-methyl-4-pyrimidin-carbonsäure) eingesetzt. Dabei enthalten die erfindungsgemäßen Zubereitungen derartige Pyrimidincarbonsäuren vorzugsweise in Mengen bis zu 15 Gew.-%. Vorzugsweise werden die Pyrimidincarbonsäuren dabei in Verhältnissen von 100:1 bis 1:100 zu den erfindungsgemäßen Verbindungen eingesetzt, wobei Verhältnisse im Bereich 1:10 bis 10:1 besonders bevorzugt sind.

Unter den Aryloximen wird vorzugsweise 2-Hydroxy-5-methyllaurophenonoxim, welches auch als HMLO, LPO oder F5 bezeichnet wird, eingesetzt. Seine Eignung zum Einsatz in kosmetischen Mitteln ist beispielsweise aus der Deutschen Offenlegungsschrift DE-A-41 16 123 bekannt. Zubereitungen, die 2-Hydroxy-5-methyllaurophenonoxim enthalten, sind demnach zur Behandlung von Hauterkrankungen, die mit Entzündungen einhergehen, geeignet. Es ist bekannt, dass derartige Zubereitungen z.B. zur Therapie der Psioriasis, unterschiedlicher Ekzemformen, irritativer und toxischer Dermatitis, UV-Dermatitis sowie weiterer allergischer und/oder entzündlicher Erkrankungen der Haut und der Hautanhangsgebilde verwendet werden können. Erfindungsgemäße Zubereitungen, die neben der Verbindung der Formel I zusätzlich eine Aryloxim, vorzugsweise 2-Hydroxy-5-methyllaurophenonoxim enthalten, zeigen überraschende antiinflammatorische Eignung. Dabei enthalten die Zubereitungen vorzugsweise 0,01 bis 10 Gew.-% des Aryloxims, wobei es insbesondere bevorzugt ist, wenn die Zubereitung 0,05 bis 5 Gew-% Aryloxim enthält.

In einer weiteren ebenfalls bevorzugten Ausführungsform der vorliegenden Erfindung enthält die Zubereitung mindestens einen Selbstbräuner.

Als vorteilhafte Selbstbräuner können unter anderem Triosen und Tetrosen, wie beispielsweise die folgenden Verbindungen, eingesetzt werden:

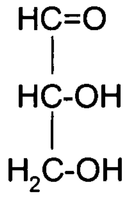 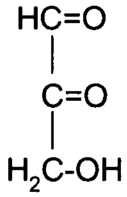 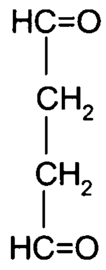 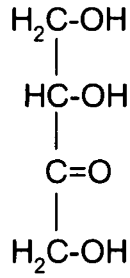

Glycerolaldehyd Hydroxymethylglyoxal $\gamma$-Dialdehyd Erythrulose

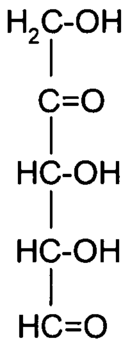 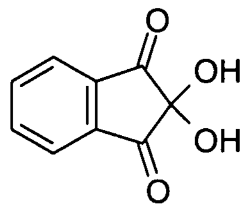

6-Aldo-D-Fructose Ninhydrin

Ferner ist das 5-Hydroxy-1,4-naphtochinon (Juglon) zu nennen, das aus den Schalen frischer Walnüsse extrahiert werden kann sowie das in den Henna-Blättern vorkommende 2-Hydroxy-1,4-naphtochinon (Lawson). Auch das Flavonoid Diosmetin und seine Glycosides oder Sulfates können eingesetzt werden. Dabei können diese Verbindungen in Form von Reinstoffen oder Pflanzenextrakten eingesetzt werden. Diosmetin kann beispielsweise vorzzugsweise in Form eines Chrysanthemum Extraktes eingesetzt werden.

Ganz besonders bevorzugt ist das 1,3-Dihydroxyaceton (DHA), ein im menschlichen Körper vorkommender dreiwertiger Zucker und dessen Derivate.

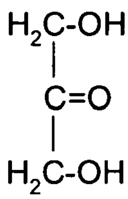

1,3-Dihydroxyaceton (DHA)

Dabei können die genannten Selbstbräuner alleine oder als Gemisch eingesetzt werden. Insbesondere bevorzugt ist es dabei, wenn DHA im Gemisch mit einem weiteren der oben genannten Selbstbräuner eingesetzt wird.

Es hat sich gezeigt, dass die Kombination von Selbstbräunern mit den erfindungsgemäßen Verbindungen im Vergleich zu der Verwendung der Selbstbräuner alleine zu einer beschleunigten Bräunung führt. Die entsprechende Verwendung der erfindungsgemäßen Verbindungen zur Beschleunigung der Bräunungswirkung von Selbstbräunern ist daher ein weiterer Gegenstand der vorliegenden Erfindung.

Alle Verbindungen oder Komponenten, die in den Zubereitungen verwendet werden können, sind entweder bekannt und käuflich erwerbbar oder können nach bekannten Verfahren synthetisiert werden.

Die eine oder die mehreren Verbindungen der Formeln Ia bis Id können in der üblichen Weise in kosmetische oder dermatologische, aber auch pharmazeutische Zubereitungen oder in Nahrungsmittel eingearbeitet werden. Geeignet sind Zubereitungen für eine äußerliche Anwendung, beispielsweise als Creme, Lotion, Gel, oder als Lösung, die auf die Haut aufgesprüht werden kann. Für eine innerliche Anwendung sind Darreichungsformeln wie Kapseln, Dragees, Pulver, Tabletten-Lösungen oder Lösungen geeignet.

Als Anwendungsform der Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays. Weitere Anwendungsformen sind z.B. Sticks, Shampoos und Duschbäder. Der Zubereitung können beliebige übliche Trägerstoffe, Hilfsstoffe und gegebenenfalls weitere Wirkstoffe zugesetzt werden.

Vorzuziehende Hilfsstoffe stammen aus der Gruppe der Konservierungsstoffe, Antioxidantien, Stabilisatoren, Lösungsvermittler, Vitamine, Färbemittel, Geruchsverbesserer.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethlyacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun-Präparate.

Zu den bevorzugten Zubereitungsformen gehören insbesondere Emulsionen.

Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:

- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fett-Ikoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäure und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexaldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder

ungesättigter, verzweigter und/oder unverzweigter Alkancarbonsäuren einer Kettenlänge von 8 bis 24, insbesondere 12-18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12}$-$_{15}$-Alkylbenzoat, Capryl-Caprinsäure-triglycerid, Dicaprylether.

Besonders vorteilhaft sind Mischungen aus $C_{12}$-$_{15}$-Alkylbenzoat und 2-Ethylhexylisostearat, Mischungen aus $C_{12}$-$_{15}$-Alkylbenzoat und Isotridecylisononanoat sowie Mischungen aus $C_{12}$-$_{15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann auch die Ölphase ferner einen Gehalt an cyclischen oder linearan Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wässrige Phase der Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere wird ein Gemisch der vorstehend genannten Lösemittel verwendet. Bei alkoholischen Lösemitteln kann Wasser ein weiterer Bestandteil sein.

Emulsionen sind vorteilhaft und enthalten z. B. die genannten Fette, Öle, Wachse und anderen Fettkörper, sowie Wasser und einen Emulgator, wie er üblicherweise für einen solchen Typ der Formuierung verwendet wird.

In einer bevorzugten Ausführungsform enthalten die Zubereitungen hydrophile Tenside.

Die hydrophilen Tenside werden bevorzugt gewählt aus der Gruppe der Alkylglucoside, der Acyllactylate, der Betaine sowie der Cocoamphoacetate.

Die Alkylglucoside werden ihrerseits vorteilhaft gewählt aus der Gruppe der Alkylglucoside, welche sich durch die Strukturformel

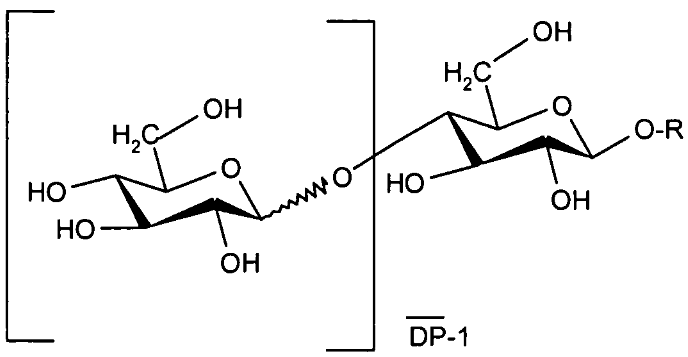

auszeichnen, wobei R einen verzweigten oder unverzweigten Alkylrest mit 4 bis 24 Kohlenstoffatomen darstellt und wobei DP einen mittleren Glucosylierungsgrad von bis zu 2 bedeutet.

Der Wert DP repräsentiert den Glucosidierungsgrad der erfindungsgemäß verwendeten Alkylglucoside und ist definiert als

$$\overline{DP} = \frac{p_1}{100}\cdot 1 + \frac{p_2}{100}\cdot 2 + \frac{p_3}{100}\cdot 3 + \ldots = \sum \frac{p_i}{100}\cdot i$$

Dabei stellen $p_1$, $p_2$, $p_3$ ... bzw. $p_i$ den Anteil der einfach, zweifach dreifach ... i-fach glucosylierten Produkte in Gewichtsprozenten dar. Erfindungsemäß vorteilhaft werden Produkte mit Glucosylierungsgraden von 1-2, insbesondere vorteilhaft von 1, 1 bis 1,5, ganz besonders vorteilhaft von 1,2-1,4, insbesondere von 1,3 gewählt.

Der Wert DP trägt den Umstande Rechnung, dass Alkylglucoside herstellungsedingt in der Regel Gemische aus Mono- und Oligoglucosiden darstellen. Erfindungsgemäß vorteilhaft ist ein relativ hoher Gehalt an Monoglucosiden, typischerweise in der Größenordnung von 40-70 Gew.-%.

Besonders vorteilhaft verwendete Alkylglylcoside werden gewählt aus der Gruppe Octylglucopyranosid, Nonylglucopyranosid, Decylglucopyranosid, Undecylglucopyranosid, Dodecylglucopyranosid, Tetradecylglucopyranosid und Hexadecylglucopyranosid.

Es ist ebenfalls von Vorteil, natürliche oder synthetische Roh- und Hilfsstoffe bzw. Gemische einzusetzen, welche sich durch einen wirksamen Gehalt an den erfindungsgemäß verwendeten Wirkstoffen auszeichnen, beispielsweise Plantaren® 1200 (Henkel KGaA), Oramix® NS 10 (Seppic).

Die Acyllactylate werden ihrerseits vorteilhaft gewählt aus der Gruppe der Substanzen, welche sich durch die Strukturformel

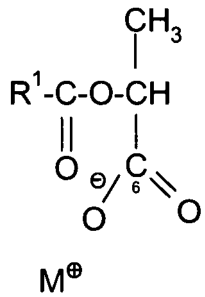

auszeichnen, wobei $R^1$ einen verzweigten oder unverzweigten Alkylrest mit 1 bis 30 Kohlenstoffatomen bedeutet und $M^+$ aus der Gruppe der Alkaliionen sowie der Gruppe der mit einer oder mehreren Alkyl- und/oder mit einer oder mehreren Hydroxyalkylresten substituierten Ammoniumionen gewählt wird bzw. dem halben Äquivalent eines Erdalkalions entspricht.

Vorteilhaft ist beispielsweise Natriumisostearyllactylat, beispielsweise das Produkt Pathionic® ISL von der Gesellschaft American Ingredients Company.

Die Betaine werden vorteilhaft gewählt aus der Gruppe der Substanzen, welche sich durch die Strukturformel

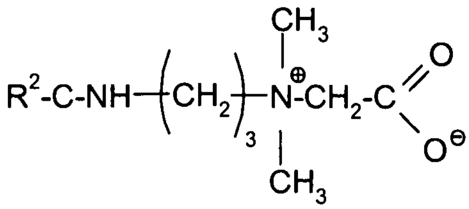

auszeichnen, wobei $R^2$ einen verzweigten oder unverzweigten Alkylrest mit 1 bis 30 Kohlenstoffatomen bedeutet.

Insbesondere vorteilhaft bedeutet $R^2$ einen verzweigten oder unverzweigten Alkylrest mit 6 bis 12 Kohlenstoffatomen.

Vorteilhaft ist beispielsweise Capramidopropylbetain, beispielsweise das Produkt Tego® Betain 810 von der Gesellschaft Th. Goldschmidt AG.

Als vorteilhaftes Cocoamphoacetat wird beispielsweise Natriumcocoamphoacetat gewählt, wie es unter der Bezeichnung Miranol® Ultra C32 von der Gesellschaft Miranol Chemical Corp. erhältlich ist.

Die Zubereitungen sind vorteilhaft dadurch gekennzeichnet, dass das oder die hydrophilen Tenside in Konzentrationen von 0,01-20 Gew.-% bevorzugt 0,05-10 Gew.-%, besonders bevorzugt 0,1-5 Gew.-%, jeweils bezogen auf das Gesamtgewicht der Zusammensetzung, vorliegt oder vorliegen.

Zu Anwendung werden die kosmetischen und dermatologischen Zubereitungen in der für Kosmetika üblichen Weise auf die Haut und/oder die Haare in ausreichender Menge aufgebracht.

Kosmetische und dermatologische Zubereitungen enthaltend mindestens eine Verbindung der Formeln Ia bis Id können in verschiedenen Formen vorliegen. So können sie z.B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Waser-in-Öl-in-Wasser (W/O/W), ein Gel, einen festen Stift, eine Salbe oder auch ein Aerosol darstellen. Es ist auch vorteilhaft, Wirkstoffe in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt. Bevorzugt werden Emulsionen. O/W-Emulsionen werden besonders bevorzugt. Emulsionen, W/O-Emulsionen und O/W-Emulsionen sind in üblicher Weise erhältlich.

Als Emulgatoren können beispielsweise die bekannten W/O- und O/W-Emulgatoren verwendet werden. Es ist vorteilhaft, weitere übliche Co-emulgatoren in den erfindungsgemäßen bevorzugten O/W-Emulsionen zu verwenden.

Erfindungsgemäß vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkhole, Cetylalkohole, Cetylstearylalkohole (Cetearylalkohole) zu wählen. Insbesondere bevorzugt sind: Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)-stearylether (Steareth-17),Polyethylenglycol(18)stearylether (Steareth-18), Polyethylenglycol(19)stearylether (Steareth-19), Polyethylenglycol(20)-stearylether (Steareth-20), Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)-isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol(20)isostearylether (Isosteareth-20), Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)-cetylether (Ceteth-18), Polyethylenglycol(19)cetylether (Ceteth-19), Polyethylen-glycol(20)cetylether (Ceteth-20), Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)isocetylether (Isoceteth-16), Polyethylenglycol(17)-isocetylether (Isoceteth-17), Polyethylenglycol(18)isocetylether (Isoceteth-18), Polyethylenglycol(19)isocetylether (Isoceteth-19), Polyethylenglycol(20)isocetylether (Isoceteth-20), Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15), Polyethylenglycol(12)laurylether (Laureth-12), Polyethylenglycol(12)-isolaurylether (Isolaureth-12), Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)-cetylstearylether (Ceteareth-17), Polyethylenglycol(18)cetylstearylether (Ceteareth-18), Polyethylenglycol(19)cetylstearylether (Ceteareth-19), Polyethylenglycol(20)cetylstearylether (Ceteareth-20).

Es ist ferner von Vorteil, die Fettsäureethoxylate ausfolgender Gruppe zu wählen:

Polyethylenglycol(20)stearat, Polyethylenglycol(21)stearat,
Polyethylenglycol(22)stearat, Polyethylenglycol(23)stearat,
Polyethylenglycol(24)stearat, Polyethylenglycol(25)stearat,
Polyethylenglycol(12)isostearat, Polyethylenglycol(13)isostearat,
Polyethylenglycol(14)isostearat, Polyethylenglycol(15)isostearat,
Polyethylenglycol(16)isostearat, Polyethylenglycol(17)isostearat,
Polyethylenglycol(18)isostearat, Polyethylenglycol(19)isostearat,
Polyethylenglycol(20)isostearat, Polyethylenglycol(21)isostearat,
Polyethylenglycol(22)isostearat, Polyethylenglycol(23)isostearat,
Polyethylenglycol(24)isostearat, Polyethylenglycol(25)isostearat,
Polyethylenglycol(12)oleat, Polyethylenglycol(13)oleat,
Polyethylenglycol(14)oleat, Polyethylenglycol(15)oleat,
Polyethylenglycol(16)oleat, Polyethylenglycol(17)oleat,
Polyethylenglycol(18)oleat, Polyethylenglycol(19)oleat,
Polyethylenglycol(20)oleat,

Als ethoxylierte Alkylethercarbonsäure bzw. deren Salz kann vorteilhaft das Natriumlaureth-11-carboxylat verwendet werden. Als Alkylethersulfat kann Natrium Laureth1-4sulfat vorteilhaft verwendet werden. Als ethoxyliertes Cholesterinderivat kann vorteilhaft Polyethylenglycol(30)Cholesterylether verwendet werden. Auch Polyethylenglycol(25)Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/cprinat, Polyethylenglycol-(20)glyceryloleat, Polyethylenglycol(20)glycerylisostearat, Polyethylenglycol(18)glyceryloleat(cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als fakultative, dennoch erfindungsgemäß gegebenenfalls vorteilhafte W/O-Emulgatoren können eingesetzt werden:

Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C-Atome, Diglycerinester gesättigter und/oder ungesättigter, verzweigter und/oder unverzweigter Alkancarbonsäuren einer Kettenlänge von 8 bis 24, insbesondere 12-18 C-Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkhole einer Kettenlänge von 8 bis 24, insbesondere 12-18 C-Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Bevorzugte Zubereitungen eignen sich besonders zum Schutz menschlicher Haut gegen Alterungsprozesse sowie vor oxidativem Stress, d.h. gegen Schädigungen durch Radikale, wie sie z.B. durch Sonneneinstrahlung, Wärme oder andere Einflüsse erzeugt werden. Dabei liegt sie in verschiedenen, für diese Anwendung üblicherweise verwendeten Darreichungsformen vor. So kann sie insbesondere als Lotion oder Emulsion, wie als Creme oder Milch (O/W, W/O, O/W/O, W/O/W), in Form ölig-alkoholischer, ölig-wässriger oder wässrigalkoholischer Gele bzw. Lösungen, als feste Stifte vorliegen oder als Aerosol konfektioniert sein.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -milch vorliegt und außer der oder den erfindungsgemäßen Verbindungen beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die erfindungsgemäße Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren,

Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane.

Die kosmetische Zubereitung kann auch zum Schutz der Haare gegen fotochemische Schäden verwendet werden, um Veränderungen von Farbnuancen, ein Entfärben oder Schäden mechanischer Art zu verhindern. In diesem Fall erfolgt geeignet eine Konfektionierung als Shampoo, Lotion, Gel oder Emulsion zum Ausspülen, wobei die jeweilige Zubereitung vor oder nach dem Shampoonieren, vor oder nach dem Färben oder Entfärben bzw. vor oder nach der Dauerwelle aufgetragen wird. Es kann auch eine Zubereitung als Lotion oder Gel zum Frisieren und Behandeln, als Lotion oder Gel zum Bürsten oder Legen einer Wasserwelle, als Haarlack, Dauerwellenmittel, Färbe- oder Entfärbemittel der Haare gewählt werden. Die Zubereitung mit Lichtschutzeigenschaften kann außer der oder den erfindungsgemäßen Verbindungen verschiedene, in diesem Mitteltyp verwendete Adjuvantien enthalten, wie Grenzflächen aktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Weitere Gegenstände der vorliegenden Erfindung sind ein Verfahren zur Herstellung einer Zubereitung, welches dadurch gekennzeichnet ist, dass mindestens eine Verbindung der Formel Ia, Ib, Ic oder Id mit einem kosmetisch oder dermatologisch oder für Nahrungsmittel oder für Haushaltsprodukte geeigneten Träger vermischt wird, und die Verwendung einer Verbindung der Formel Ia, Ib, Ic oder Id zur Herstellung einer Zubereitung mit antioxidanten Eigenschaften.

Die Zubereitungen können dabei mit Hilfe von Techniken hergestellt werden, die dem Fachmann wohl bekannt sind.

Das Vermischen kann ein Lösen, Emulgieren oder Dispergieren der erfindungsgemäßen Verbindung in dem Träger zur Folge haben.

In einem erfindungsgemäß bevorzugten Verfahren wird die Verbindung nach Formeln Ia bis Id hergestellt durch Hydrierung mindestens einer Verbindung der Formeln Ia bis Id en

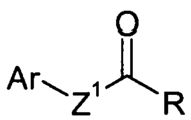

I en

wobei die Reste Ar, $Z^1$ und R denen der gewünschten Formeln Ia bis Id entsprechen, hydriert wird.

Analoges gilt für die Hydrierung der Verbindungen der Formel la en, Ib en und Id en zu den Verbindungen der Formeln la bis Id.

Zur Hydrierung ist z. B. molekularer Wasserstoff geeignet. Wenn molekularer Wasserstoff für die Hydrierung der Verbindungen der Formel I en verwendet wird, geschieht die Hydrierung vorzugsweise in Gegenwart eines Katalysators bzw. Katalysatorsystems.

Als Katalysatoren für die Hydrierung eignen sich alle gängigen homogenen und heterogenen Katalysatoren, insbesondere bevorzugt wird als Katalysator mindestens ein Edelmetall vorzugsweise ausgewählt aus den Elementen Pt, Pd und Rh, oder ein Übergangsmetall, wie Mo, W, Cr, besonders aber Fe, Co und Ni, entweder einzeln oder im Gemisch eingesetzt. Dabei können der oder die Katalysatoren oder Katalysatorgemische auch auf Trägern wie Kohle, Aktivkohle, Aluminiumoxid, Bariumcarbonat, Bariumsulfat, Calciumcarbonat, Strontiumcarbonat oder Kieselgur eingesetzt werden. Dabei kann das Metall auch in Form der Raney-Verbindung, beispielsweise Raney-Nickel eingesetzt werden. Wird die Katalyse in einem homogenen Verfahren durchgeführt, so ist es bevorzugt, wenn als Katalysator eine oder mehrere Komplexverbindungen der genannten Metalle, wie beispielsweise der Wilkinson-Katalysator [Chlor-tris(triphenylphosphin)rhodium], eingesetzt wird. Es können ferner Salze der genannten Metalle eingesetzt werden, die in situ durch ein Reduktionsmittel reduziert werden können und in situ eine fein verteilte Metall (0)-Spezies erzeugen. Geeignete Edelmetallsalze sind beispielsweise Palladiumacetat, Palladiumbromid oder Palladiumchlorid, geeignete Reduktionsmittel sind beispielsweise Wasserstoff, Hydrazin, Natriumborhydrid oder Formiate. In einer bevorzugten Variante der vorliegenden Erfindung wir ein heterogener Katalysator eingesetzt, wobei es insbesondere bevorzugt ist als Katalysator bei dem erfindungsgemäßen Verfahren Pd oder Pt, vorzugsweise auf Aktivkohleträger, beispielsweise 5 Gew.-% Pd oder Pt auf C, einzusetzen.

Die Hydrierung wird üblicherweise bei einer Temperatur im Bereich von 20 - 150°C durchgeführt. Weiter wird die Hydrierung vorteilhaft bei einem Wasserstoff-Druck von 1 bis 200 bar durchgeführt.

Als Lösungsmittel eignen sich protische Lösungsmittel, insbesondere die dem Fachmann bekannten üblichen protischen Lösungsmittel, wie Wasser, niedere Alkohole, wie beispielsweise Methanol, Ethanol und Isopropanol, sowie primäre und sekundäre Amine und Gemische solcher protischer Lösungsmittel, wobei es insbesondere bevorzugt sein kann, wenn als Lösungsmittel Wasser eingesetzt wird.

Als Lösungsmittel für diese Umsetzung eignen sich weiter auch übliche aprotische Lösungsmittel. Beispielsweise können Diethylether, Tetrahydrofuran, Benzol, Toluol, Acetonitril, Dimethoxyethan, Dimethylformamid, Dimethylsulfoxid und N-Methyl-pyrrolidon eingesetzt werden.

In einer ebenfalls bevorzugten Ausführungsform des erfindungsgemäßen Herstellverfahrens wird die Hydrierung in Substanz durchgeführt, d.h. es ist kein zusätzliches Lösungsmittel erforderlich.

Nach beendeter Reaktion kann die Aufarbeitung nach üblichen Methoden erfolgen. Beispielsweise kann der Katalysator abfiltriert, das Filtrat vom Lösungsmittel befreit werden, z. B. durch Erhitzen bei im Vergleich zu Atmosphärendruck erniedrigtem Druck und das so erhaltene Produkt durch übliche Methoden weiter aufgereinigt werden.

Die weitere Aufreinigung der Reaktionsprodukte kann ebenfalls durch übliche Methoden, beispielsweise durch Umkristallisieren aus einem geeigneten Lösungsmittel, oder durch chromatographische Methoden erfolgen.

Ein weiterer Gegenstand der Erfindung ist ein Verfahren zur Herstellung einer Verbindung der Formel Ib oder Ic

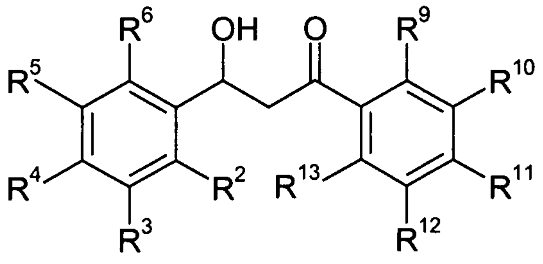

Ib

,

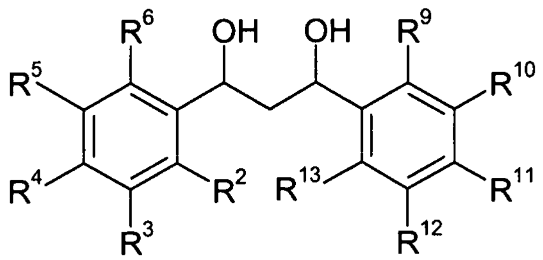

Ic

,

wobei

$R^4$ und $R^{11}$ jeweils unabhängig voneinander H, geradkettige oder verzweigte $C_1$- bis $C_{20}$-Alkylgruppe, geradkettige oder verzweigte $C_1$- bis $C_{20}$-Alkoxygruppe oder geradkettige oder verzweigte $C_1$- bis $C_{20}$-Dialkylaminogruppe,
$R^6$ H oder eine Carbonsäure-, Phosphorsäure-, Sulfonsäure-, Schwefelsäure- oder Sulfonfunktion, die mit geradkettigen oder verzweigten $C_1$- bis $C_{20}$-Alkylgruppen oder geradkettigen oder verzweigten $C_3$- bis $C_{20}$-Alkenylgruppen verestert oder alkyliert sein kann und
$R^2$, $R^3$, $R^5$, $R^9$, $R^{10}$, $R^{12}$ und $R^{13}$ H bedeuten, dadurch gekennzeichnet, dass eine Verbindung der Formel Ib en

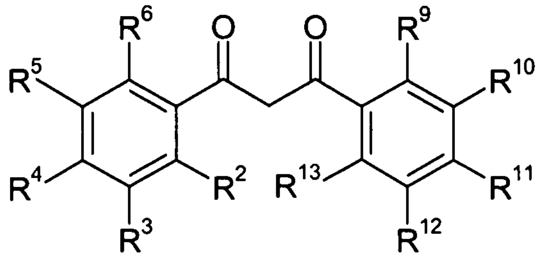

Ib en

wobei die Reste $R^2$ bis $R^6$ und $R^9$ bis $R^{13}$ eine zuvor genannte Bedeutung haben, hydriert wird.

Die Verbindungen der Formeln la bis Id können jedoch auch über Kupplungsreaktionen hergestellt werden, wie sie in spezieller Form in den Beispielen offenbart sind. Eine generelle Übertragbarkeit dieser Synthesen, d.h. eine Reaktion eines entsprechend substituierten Aryl-aldehyds mit einem entsprechend substituierten Arylketons ist dem Synthesefachmann geläufig.

Allgemeines Schema:

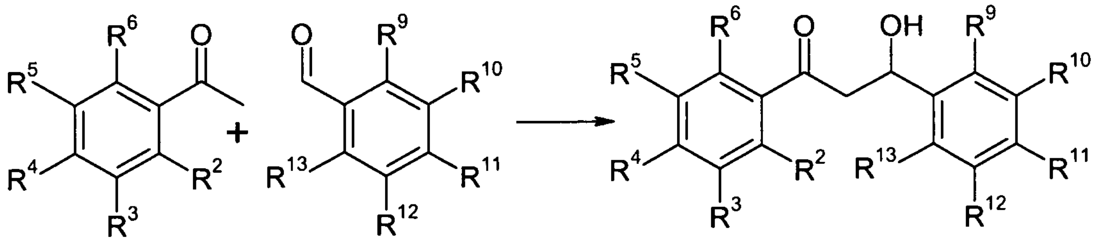

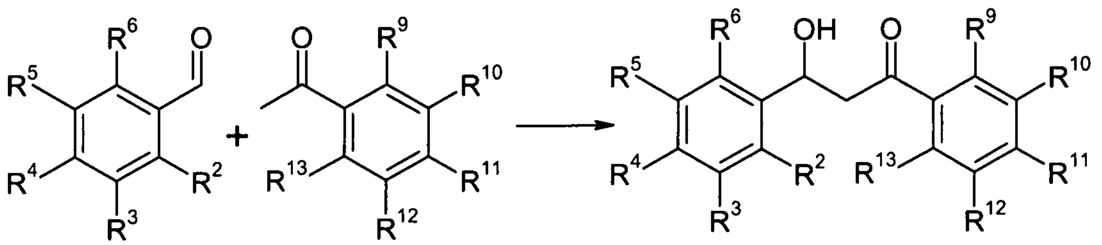

Es wurde auch festgestellt, dass Verbindungen der Formeln Ia bis Id, stabilisierend auf die Zubereitung wirken können. Bei der Verwendung in entsprechenden Produkten bleiben diese daher auch länger stabil und verändern ihre galenische und sensorische Beschaffenheit nicht. Insbesondere bleibt auch bei längerdauernder Anwendung bzw. längerer Lagerung die Wirksamkeit der Inhaltsstoffe, z.B. Vitamine, erhalten. Dies ist unter anderem besonders vorteilhaft bei Zusammensetzungen zum Schutz der Haut gegen die Einwirkung von UV-Strahlen, da diese Kosmetika besonders hohen Belastungen durch die UV-Strahlung ausgesetzt sind.

Die positiven Wirkungen von Verbindungen der Formeln Ia bis Id ergeben deren besondere Eignung zur Verwendung in kosmetischen oder pharmazeutischen Zubereitungen.

Ebenso positiv sind die Eigenschaften von Verbindungen mit der Formel Ia, Ib, Ic oder Id zu werten für eine Verwendung in Nahrungsmitteln oder als Nahrungsergänzungsmittel oder als "functional food". Die weiteren zu Nahrungsmitteln ausgeführten Erläuterungen gelten sinngemäß auch für Nahrungsergänzungsmittel und für "functional food".

Die Nahrungsmittel, die mit einer oder mehreren Verbindungen Formel Ia, Ib, Ic oder Id der Formeln Ia bis Id angereichert werden können, umfassen alle Materialien, die für den Verzehr durch Tiere oder für den Verzehr durch Menschen geeignet sind, beispielsweise Vitamine und Provitamine davon, Fette, Mineralien oder Aminosäuren ". (Die Nahrungsmittel können fest sein aber auch flüssig, also als Getränk vorliegen).

Weitere Gegenstände der vorliegenden Erfindung sind dementsprechend die Verwendung mindestens einer Verbindung nach Formel Ia, Ib, Ic oder Id als Nahrungsmittelzusatz für die human- oder Tierernährung sowie Zubereitungen, die Nahrungsmittel oder Nahrungsergänzungsmittel sind und entsprechende Träger enthalten.

Nahrungsmittel, die mit einer oder mehreren Verbindungen der Formel Ia, Ib, Ic oder Id angereichert werden können, sind beispielsweise auch Nahrungsmittel, die aus einer einzigen natürlichen Quelle stammen, wie z.B. Zucker, ungesüßter Saft, Nektar oder Püree von einer einzigen Pflanzenspezies, wie z.B. ungesüßter Apfelsaft (z.B. auch eine Mischung verschiedener Sorten Apfelsaft), Grapefruitsaft, Orangensaft, Apfelkompott, Aprikosennektar, Tomatensaft, Tomatensoße, Tomatenpüree usw.

Weitere Beispiele für Nahrungsmittel, die nach der vorliegenden Erfindung mit einer oder mehreren Verbindungen der Formel Ia, Ib, Ic oder Id angereichert werden können, sind Korn oder Getreide einer einzigen Pflanzenspezies und Materialien, die aus derartigen Pflanzenspezies hergestellt werden, wie z.B. Getreidesirup, Roggenmehl, Weizenmehl oder Haferkleie. Auch Mischungen von derartigen Nahrungsmitteln sind geeignet, um mit einer oder mehreren Verbindungen der Formel Ia, Ib, Ic oder Id angereichert zu werden, beispielsweise Multivitaminpräparate, Mineralstoffmischungen oder gezuckerter Saft. Als weitere Beispiele für Nahrungsmittel, die nach der vorliegenden Erfindung mit einer oder mehreren Verbindungen der Formel Ia bis Id angereichert werden können, seien Nahrungsmittelzubereitungen, beispielsweise zubereitete Cerealien, Gebäck, Mischgetränke, speziell für Kinder zubereitete Nahrungsmittel, wie Joghurt, Diätnahrungsmittel, kalorienarme Nahrungsmittel oder Tierfutter, genannt.

Die Nahrungsmittel, die nach der vorliegenden Erfindung mit einer oder mehreren Verbindungen der Formel Ia, Ib, Ic oder Id angereichert werden können, umfassen somit alle genießbaren Kombinationen von Kohlenhydraten, Lipiden, Proteinen, anorganischen Elementen, Spurenelementen, Vitaminen, Wasser oder aktiven Metaboliten von

Pflanzen und Tieren.

Die Nahrungsmittel, die nach der vorliegenden Erfindung mit einer oder mehreren Verbindungen der Formel Ia, Ib, Ic oder Id angereichert werden können, werden vorzugsweise oral angewendet, z.B. in Form von Speisen, Pillen, Tabletten, Kapseln, Pulver, Sirup, Lösungen oder Suspensionen.

Die mit einer oder mehreren Verbindungen der Formel Ia, Ib, Ic oder Id angereicherten erfindungsgemäßen Nahrungsmittel können mit Hilfe von Techniken hergestellt werden, die dem Fachmann wohl bekannt sind.

Durch ihre Wirkung als Antioxidationsmittel bzw. als Radikalfänger eignen sich die Verbindungen der Formel Ia, Ib, Ic oder Id auch als Arzneimittelinhaltsstoff. Sie wirken dabei unterstützend oder substituierend zu natürlichen Mechanismen, welche Radikale im Körper abfangen. Die erfindungsgemäßen Verbindungen können in ihrer Wirkung teilweise mit Radikalfängern wie Vitamin C verglichen werden. Die Verbindungen der Formel Ia, Ib, Ic oder Id können beispielsweise zur vorbeugenden Behandlung von Entzündungen und Allergien der Haut sowie in bestimmten Fällen zur Verhütung bestimmter Krebsarten verwendet werden. Insbesondere eignen sich erfindungsgemäßen Verbindungen zur Herstellung eines Arzneimittels zur Behandlung von Entzündungen, Allergien und Irritationen, insbesondere der Haut. Ferner können Arzneimittel hergestellt werden in einer Wirkung als Venentonikum, als Mittel zur Erhöhung der Festigkeit von Blutkapillaren, als Hemmstoff für Cuperose, als Hemmstoff chemischer, physikalischer oder aktinischer Erytheme, als Mittel zur Behandlung empfindlicher Haut, als Dekongestionsmittel, als Entwässerungsmittel, als Mittel zum Schlankmachen, als Antifaltenmittel, als Stimulatoren der Synthese von Komponenten der extrazellulären Matrix, als stärkendes Mittel zur Verbesserung der Hautelastizität und als Antialterungsmittel. Weiter zeigen in diesem Zusammenhang bevorzugte erfindungsgemäßen Verbindungen antiallergische und antiinflammatorische und antiirritative Wirkungen. Sie eignen sich daher zur Herstellung von Arzneimitteln zur Behandlung von Entzündungen oder allergischen Reaktionen.

Im folgenden wird die Erfindung anhand von Beispielen näher erläutert die Erfindung ist im gesamten beanspruchten Bereich ausführbar und nicht auf die hier genannten Beispiele beschränkt.

## Beispiele

### Beispiel 1: Herstellung von 2-(Hydroxy-phenyl-methyl)-5-methoxy-phenol

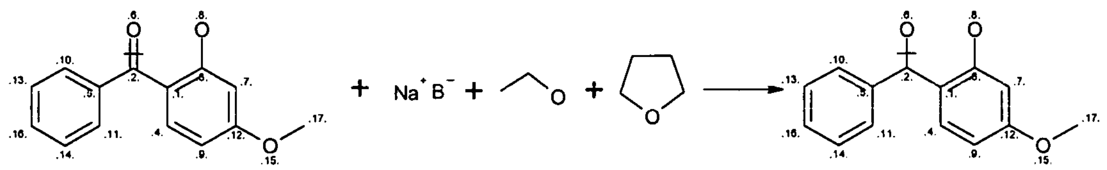

Natriumborhydrid wird in 75 ml Ethanol gelöst und das in 5 ml THF gelöste Edukt (2-Hydroxy-4-methoxyphenyl)-phenylmethanon innerhalb von ca. 10 min zugetropft. Das Reaktionsgemisch wird 1 h bis zum vollständigen Verbrauch von Natriumborhydrid bei Raumtemperatur gerührt. Das Lösungsmittel wird im Vakuum entfernt. Der Rückstand wird in Methyl-t-butylether (MTBE)/Wasser verteilt und die wässrige Phase noch 3 mal mit 50 ml MTBE extrahiert. Die vereinigten organischen Phasen werden über Natriumsulfat getrocknet und das Lösungsmittel wird im Vakkum entfernt.

### Beispiel 2: Herstellung von 2-[(4-Diethylamino-2-hydroxy-phenyl)-hydroxy-methyl]-benzoesäure-hexylester (2 alternative Routen)

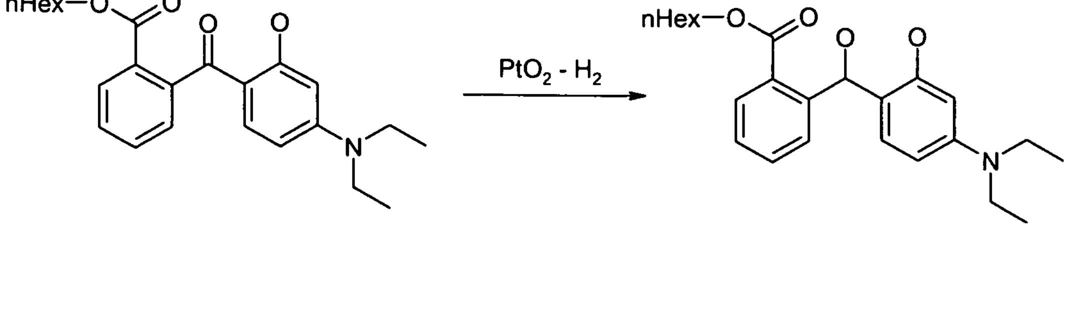

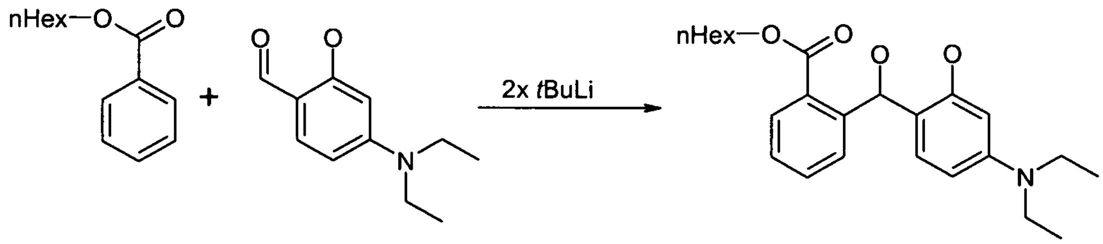


Analog Beispiel 1 bzw. 2 können prinzipiell alle Verbindungen der Formel I hergestellt werden. Beispielsweise können die folgenden Alkohole aus den entsprechenden Kentonen erhalten werden:

2-(Hydroxy-phenyl-methyl)-5-diethylamino-phenol
2-(Hydroxy-phenyl-methyl)-phenol
2-(Hydroxy-phenyl-methyl)-5-sulfo-phenol
2-(Hydroxy-phenyl-methyl)-5-methoxy-4-sulfo-phenol
2-[(4-Diethylamino-2-hydroxy-phenyl)-hydroxy-methyl]-benzoesäure-ethylester
2-[(4-Diethylamino-2-hydroxy-phenyl)-hydroxy-methyl]-benzoesäure-ethylhexylester
2-[(2-hydroxy-phenyl)-hydroxy-methyl]-benzoesäure-ethylhexylester
3-Hydroxy-1,3-diphenyl-propan-1-on
1-(4-tert-butyl-phenyl)-3-hydroxy-3-phenyl-propan-1-on
1-(4-tert-butyl-phenyl)-3-hydroxy-3-(4-methoxy-phenyl)-propan-1-on
3-(4-tert-butyl-phenyl)-3-hydroxy-1-phenyl-propan-1 -on
3-(4-tert-butyl-phenyl)-3-hydroxy-1-(4-methoxy-phenyl)-propan-1-on

**Beispiel 3:**

Synthese von 3-(4-Methoxy-phenyl)-3-hydroxy-1-(4-tert-butyl-phenyl)-propan-1-on

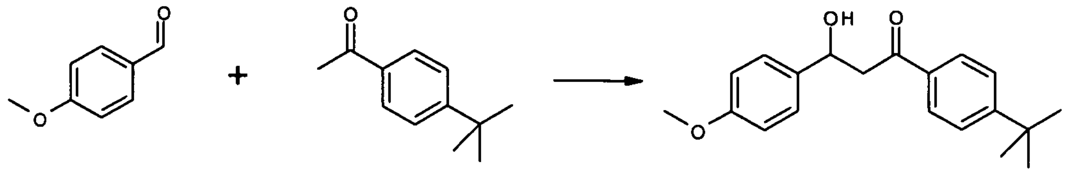

Zu einer Lösung von p-tert-Butylacetophenon (500 mg, 2,8 mmol) in THF (15 mL) werden bei -78°C unter Stickstoff 3,4 ml LDA (Lithiumdiisopropylamid) 1 M in THF (3,4 mmol) zugesetzt. Das Gemisch wird für 1 h gerührt bevor man 5 mL einer THF-Lösung von p-Methoxybenzaldehyd (425 mg, 3,1 mmol) zusetzt. Innerhalb von 1 h läßt man die Reaktion -40°C erreichen. Zum Reaktionsgemisch wird anschließend eine kalte wäßrige Lösung von $NH_4Cl$ 1 N gegeben. Das resultierende Gemisch wird einer Verteilung über Wasser (30 mL) und Dichlormethan (30 mL) unterzogen. Die resultierende wässrige Phase wird anschließend mit Dichlormethan extrahiert (2x 30 mL). Die vereinigten organischen Phasen trocknet man über $MgSO_4$. Nach Entfernen des Lösungsmittels erhält man 3-(4-Methoxy-phenyl)-3-hydroxy-1-(4-tert-butyl-phenyl)-propan-1-on.

**Beispiel 4:**

Synthese von 3-(4-tert-Butyl-phenyl)-3-hydroxy-1-(4-methoxy-phenyl)-propan-1-on

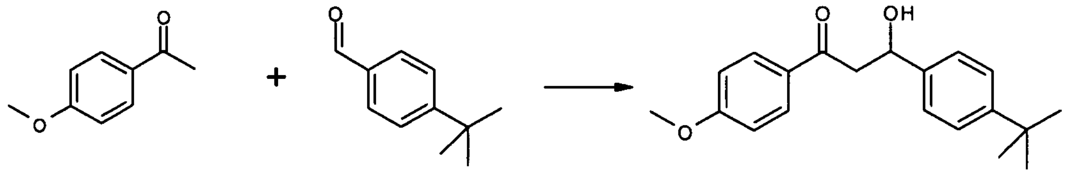

Zu einer Lösung von p-Methoxyacetophenon (500 mg, 3,3 mmol) in THF (Tetrahydrofuran) (15 mL) werden bei -78°C unter Stickstoff 4,0 ml LDA 1M in THF (4,0 mmol) zugesetzt. Das Gemisch wird für 1 h gerührt bevor man 5 mL einer THF-Lösung von p-tert-butyl-Benzaldehyd (594 mg, 3,7 mmol) zusetzt. Innerhalb von 1 h läßt man die Reaktion -40°C erreichen. Zum Reaktionsgemisch wird anschließend eine kalte wässrige Lösung von $NH_4Cl$ 1 N gegeben. Das resultierende Gemisch wird einer Verteilung über Wasser (30 mL) und Dichlormethan (30 mL) unterzogen. Die resultierende wässrige Phase wird anschließend mit Dichlormethan extrahiert (2x 30 mL). Die vereinigten organischen Phasen trocknet man über $MgSO_4$. Nach Entfernen des Lösungsmittels erhält man 3-(4-tert-Butyl-phenyl)-3-hydroxy-1-(4-methoxy-phenyl)-propan-1-on.

**Beispiel 5: Oxidation im UV Licht**

Bei Bestrahlung mittels UV-Licht ändert sich das UV-Spektrums der erfindungsgemäßen Verbindungen gemäß Beispiel 1 bzw. 2.

Die Bestrahlung kann dabei mittels einer Atlas Sun Test CPS, Xenon Lamp mit UV-Spezialglas-Filter bei einer Leistung von 95,69 $W/m^2$ im Bereich 290 - 400 nm erfolgen.

Bereits nach etwa 60 min zeigt sich eine deutlich erhöhte UV Absorption der Verbindungen, die bei längerer Bestrahlung weiter zunimmt.

**Beispiel 5a: Oxidation im UV Licht in Gegenwart weiterer Antioxidantien**

Emulsionen enthaltend Verbindungen gemäß Beispiel 1 oder 2 und beta-Carotin zeigen bei Bestrahlung mit UV Licht im Vergleich zu solchen, die lediglich beta-Carotin enthalten, dass die Absorption des beta-Carotin ($E_{max}$ im Bereich 440-480 nm) in den erfindungsgemäßen Proben deutlich stärker ist. Folglich ist der beta-Carotin Abbau in der erfindungsgemäßen Emulsion reduziert; die erfindungsgemäßen Verbindungen stabilisieren das beta-Carotin.

**Beispiel 5b: DPPH-Assay**

Die radikalreduzierende Wirkung der erfindungsgemäßen Verbindungen kann beispielsweise mit dem 2,2-Diphenyl-1-picrylhydrazyl(DPPH)-Assay gezeigt werden. 2,2-Diphenyl-1-picrylhydrazyl ist ein in Lösung stabiles freies Radikal. Das ungepaarte Elektron führt zu einer starken Absorptionsbande bei 515 nm, die Lösung ist dunkel-violett gefärbt. In Gegenwart eines Radikalfängers wird das Elektron gepaart, die Absorption verschwindet und die Entfärbung verläuft stöchiometrisch unter Berücksichtigung der aufgenommenen Elektronen. Gemessen wird die Extinktion im Photometer. Die antiradikalische Eigenschaft der zu testenden Substanz wird bestimmt, indem man die Konzentration ermittelt, bei der 50 % des eingesetzten 2,2-Diphenyl-1-picrylhydrazyls mit dem Radikalfänger reagiert haben. Ausgedrückt wird diese Konzentration als $EC_{50}$, ein Wert, der unter den gegebenen Messbedingungen als Substanzeigenschaft zu betrachten ist. Verglichen wird die untersuchte Substanz mit einem Standard (z.B. Tocopherol). Der $EC_{50}$-Wert ist dabei ein Maß für die Kapazität der jeweiligen Verbindung Radikale zu fangen. Je niedriger der $EC_{50}$-Wert ist, desto höher ist die Kapazität Radikale zu fangen.

**Durchführung:**

Es wird eine Stammlösung von 2,2-Diphenyl-1-pikrylhydrozyl (DPPH) in Ethanol hergestellt (0,025 g/L DPPH-Radikale). Aliquots dieser Lösung werden mit verschiedenen Konzentrationen der zu testenden Verbindung versetzt. Es wird bei 515 nm, 25°C und 1 cm jeweils die Extinktion gemessen.

Als $EC_{50}$ wird der Wert ermittelt, bei dem noch 50% der ursprünglichen DPPH-radikal-Konzentration vorliegt. Je kleiner dieser Wert ist, desto höher ist die entsprechende radikalreduzierende Aktivität.

Die Reaktionszeit, die benötigt wird, um diesen Wert zu erreichen wird in dem Wert $T_{EC50}$ angegeben (in Minuten).

Eine detaillierte Beschreibung neben dem soeben beschriebenen DPPAassay findet man auch für den Lipid Assay (oder 2,2'-azobis(2-aminepropane) = ABAP assay) oder den TEAC assay (TEAC = trolox equivalent antioxidant capacity) in Bünger et al, International Journal of Cosmetic Science, 2006, 28, 135-146.

Die Daten für Verbindung (5) = 2-(Hydroxy-phenyl-methyl)-5-methoxyphenol aus Beispiel 1 sind nach der Methode aus Bünger et al sind:

DPPH assay EC50 [μmol$L^{-1}$] = 1.90 (dieser Wert ist nicht überragend) Lipid Assay: 8% (auch nicht überragend) TEAC Assay: 50% (dieser Wert ist gut)

**Beispiel 6: Photokonversionstest**

Beispiel 6a: Photokonverionstest für Verbindung (9) = (4-Methoxy-phenyl)-phenyl-methanol:

Eine 4%ige Lösung der Substanz in Isopropylmyristat wird zu 2μL$cm^{-2}$ auf einen rauhen Plexiglasträger aufgebracht. Die Probe wird für 1 h 50min im Suntester einer simulierten Sonnenbestrahlung ausgesetzt. Nach Bestrahlung wird die Probe mit 40mL Isopropanol extrahiert und auf ein exaktes Volumen von 50 mL aufgefüllt. Im Vergleich zu einer unbestrahlten Probe ergibt sich für die bestrahlte Probe eine Extinktionszunahme im Substanzmaximum bei 285nm von 0.77 Absorptionseinheiten.

Beispiel 6b: Photokonversionstest für Verbindung (5) aus Beispiel 1 = 2-(Hydroxy-phenyl-methyl)-5-methoxy-phenol

Die Substanz wird auf angerauhte Plexiglasplates aufgetragen (0,75mg/$cm^{-2}$) und unter Sonnensimulation bestrahlt. Die Dosis ist als absolute, ungewichtete Gesamt-UV-Bestrahlungsdosis aufzufassen. Nach jedem Bestrahlungsschritt wird die UV-Absorption der Probe gegen Placebo gemessen (Placebo = Plexiglasplate mit Glycerin). Es zeigt sich, dass die Substanz durch Bestrahlung an Absorptionskraft gewinnt, wobei als Ausgangswert keinerlei Absorption vorliegt (siehe Figur 1).

**Beispiel 7: Zubereitungen**

Im folgenden werden beispielhaft Rezepturen für kosmetische Zubereitungen angegeben, die Verbindungen nach Beispiel 1 bzw. 2 enthalten. Entsprechende Zubereitungen können in gleicher Weise mit allen erfindungsgemäßen Verbindungen hergestellt werden.

Im übrigen sind die INCI-Bezeichnungen der handelsüblichen Verbindungen angegeben.

UV-Pearl , OMC steht für die Zubereitung mit der INCI-Bezeichnung: Water (for EU: Aqua), Ethylhexyl Methoxycinnamate, Silica, PVP, Chlorphenesin, BHT; diese Zubereitung ist im Handel unter der Bezeichnung Eusolex®UV Pearl™OMC von der Merck KGaA, Darmstadt erhältlich.

Die anderen in den Tabellen angegebenen UV-Pearl sind jeweils analog zusammengesetzt, wobei OMC gegen die angegebenen UV-Filter ausgetauscht ist.

Tabelle 1 W/O-Emulsionen (Zahlen in Gew.-%)

| | 1-1 | 1-2 | 1-3 | 1-4 | 1-5 | 1-6 | 1-7 | 1-8 | 1-9 | 1-10 |
|---|---|---|---|---|---|---|---|---|---|---|
| Titanium dioxide | | 2 | 5 | | | | | | | 3 |
| 2-(Hydroxy-phenyl-methyl)-5-methoxy-phenol | 5 | 3 | 2 | 1 | 2 | 1 | 2 | 1 | 1 | 1 |
| Zinc oxide | | | | | | | | 5 | 2 | |
| UV-Pearl , OMC | 30 | 15 | 15 | 15 | 15 | 15 | 15 | 15 | 15 | 15 |
| Polyglyceryl-3-Dimerate | 3 | 3 | 3 | 3 | 3 | 3 | 3 | 3 | 3 | 3 |
| Cera Alba | 0,3 | 0,3 | 0,3 | 0,3 | 0,3 | 0,3 | 0,3 | 0,3 | 0,3 | 0,3 |
| Hydrogenated Castor Oil | 0,2 | 0,2 | 0,2 | 0,2 | 0,2 | 0,2 | 0,2 | 0,2 | 0,2 | 0,2 |
| Paraffinium Liquidum | 7 | 7 | 7 | 7 | 7 | 7 | 7 | 7 | 7 | 7 |
| Caprylic/Capric Triglyceride | 7 | 7 | 7 | 7 | 7 | 7 | 7 | 7 | 7 | 7 |
| Hexyl Laurate | 4 | 4 | 4 | 4 | 4 | 4 | 4 | 4 | 4 | 4 |
| PVP/Eicosene Copolymer | 2 | 2 | 2 | 2 | 2 | 2 | 2 | 2 | 2 | 2 |
| Propylene Glycol | 4 | 4 | 4 | 4 | 4 | 4 | 4 | 4 | 4 | 4 |
| Magnesium Sulfate | 0,6 | 0,6 | 0,6 | 0,6 | 0,6 | 0,6 | 0,6 | 0,6 | 0,6 | 0,6 |
| Tocopherol | 0,5 | 0,5 | 0,5 | 0,5 | 0,5 | 0,5 | 0,5 | 0,5 | 0,5 | 0,5 |
| Tocopheryl Acetate | 0,5 | 0,5 | 0,5 | 0,5 | 0,5 | 0,5 | 0,5 | 0,5 | 0,5 | 0,5 |
| Cyclomethicone | 0,5 | 0,5 | 0,5 | 0,5 | 0,5 | 0,5 | 0,5 | 0,5 | 0,5 | 0,5 |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Water | ad 100 | ad 100 | ad 100 | ad 100 | ad 100 | ad 100 | ad 100 | ad 100 | ad 100 | ad 100 |

Tabelle 1 (Fortsetzung)

| | 1-11 | 1-12 | 1-13 | 1-14 | 1-15 | 1-16 | 1-17 | 1-18 |
|---|---|---|---|---|---|---|---|---|
| Titanium dioxide | 3 | | 2 | | 3 | | 2 | 5 |
| Benzylidene malonate polysiloxane | | 1 | 0,5 | | | | | |
| 3-(4-tert-butyl-phenyl)-3-hydroxy-1-(4-methoxy-phenyl)-propan-1-on | 1 | 1 | 0,5 | | | | | |
| 2-[(4-Diethylamino-2-hydroxy-phenyl)-hydroxy-methyl]-benzoesäure-hexylester | 5 | 3 | 2 | 5 | 1 | 3 | 7 | 2 |
| Polyglyceryl-3-Dimerate | 3 | 3 | 3 | 3 | | | | |
| Cera Alba | 0,3 | 0,3 | 0,3 | 0,3 | 2 | 2 | 2 | 2 |
| Hydrogenated Castor Oil | 0,2 | 0,2 | 0,2 | 0,2 | | | | |
| Paraffinium Liquidum | 7 | 7 | 7 | 7 | | | | |
| Caprylic/Capric Triglyceride | 7 | 7 | 7 | 7 | | | | |
| Hexyl Laurate | 4 | 4 | 4 | 4 | | | | |
| PVP/Eicosene Copolymer | 2 | 2 | 2 | 2 | | | | |
| Propylene Glycol | 4 | 4 | 4 | 4 | | | | |
| Magnesium Sulfate | 0,6 | 0,6 | 0,6 | 0,6 | | | | |
| Tocopherol | 0,5 | 0,5 | 0,5 | 0,5 | | | | |
| Tocopheryl Acetate | 0,5 | 0,5 | 0,5 | 0,5 | 1 | 1 | 1 | 1 |
| Cyclomethicone | 0,5 | 0,5 | 0,5 | 0,5 | | | | |
| Propylparabene | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 |
| Dicocoyl Pentyerythrityl Citrate (and) Sorbitan Sesquioleate (and) Cera Alba (and) Aluminium Stearate | | | | | 6 | 6 | 6 | 6 |
| PEG-7 Hydrogenated Castor Oil | | | | | 1 | 1 | 1 | 1 |
| Zinc Stearate | | | | | 2 | 2 | 2 | 2 |
| Oleyl Erucate | | | | | 6 | 6 | 6 | 6 |
| Decyl Oleate | | | | | 6 | 6 | 6 | 6 |
| Dimethicone | | | | | 5 | 5 | 5 | 5 |
| Tromethamine | | | | | 1 | 1 | 1 | 1 |
| Glycerin | | | | | 5 | 5 | 5 | 5 |
| Allantoin | | | | | 0,2 | 0,2 | 0,2 | 0,2 |
| water | ad 100 | ad 100 | ad 100 | ad 100 | ad 100 | ad 100 | ad 100 | ad 100 |

Tabelle 1 (Fortsetzung)

| | 1-19 | 1-20 | 1-21 | 1-22 | 1-23 | 1-24 | 1-25 | 1-26 | 1-27 | 1-28 | 1-29 |
|---|---|---|---|---|---|---|---|---|---|---|---|
| Titanium dioxide | | 2 | 5 | | | | | | | 3 | 3 |
| Benzylidene malonate polysiloxane | | | | 1 | | | | | 1 | 1 | |
| Methylene Bis-Benztriazolyl Tetramethylbutylphenol | | | | | | 1 | 2 | 1 | | | 1 |
| Zinc oxide | | | | | | | | 5 | 2 | | |
| 3-(4-tert-butyl-phenyl)-3-hydroxy-1-(4-methoxy-phenyl)-propan-1-on | 5 | 5 | 5 | 5 | 7 | 5 | 5 | 5 | 5 | 5 | 8 |
| UV-Pearl , OCR | | 10 | | | | | | | | | 5 |
| UV-Pearl, EthylhexylDimethylPABA | | | 10 | | | | | | | | |
| 4-Hydroxy-3,5-dimethoxy-benzyl-malonsäure-di 2-ethyl-hexylester | 2 | 4 | 5 | 6 | 3 | 1 | 6 | 10 | 1 | 2 | 5 |
| UV-Pearl, Homosalate, BP-3 | | | | | | | | | 10 | | |
| UV-Pearl, Ethylhexyl salicylate, BP-3 | | | | | | | | | | 10 | |
| BMDBM | | | | | | | | | | | 2 |
| UV-Pearl OMC, 4-Methylbenzylidene Camphor | 25 | | | | | | | | | | |
| Polyglyceryl-3-Dimerate | 3 | 3 | 3 | 3 | 3 | 3 | 3 | 3 | 3 | 3 | 3 |
| Cera Alba | 0,3 | 0,3 | 0,3 | 0,3 | 0,3 | 0,3 | 0,3 | 0,3 | 0,3 | 0,3 | 0,3 |
| Hydrogenated Castor Oil | 0,2 | 0,2 | 0,2 | 0,2 | 0,2 | 0,2 | 0,2 | 0,2 | 0,2 | 0,2 | 0,2 |
| Paraffinium Liquidum | 7 | 7 | 7 | 7 | 7 | 7 | 7 | 7 | 7 | 7 | 7 |
| Caprylic/Capric Triglyceride | 7 | 7 | 7 | 7 | 7 | 7 | 7 | 7 | 7 | 7 | 7 |
| Hexyl Laurate | 4 | 4 | 4 | 4 | 4 | 4 | 4 | 4 | 4 | 4 | 4 |
| PVP/Eicosene Copolymer | 2 | 2 | 2 | 2 | 2 | 2 | 2 | 2 | 2 | 2 | 2 |
| Propylene Glycol | 4 | 4 | 4 | 4 | 4 | 4 | 4 | 4 | 4 | 4 | 4 |
| Magnesium Sulfate | 0,6 | 0,6 | 0,6 | 0,6 | 0,6 | 0,6 | 0,6 | 0,6 | 0,6 | 0,6 | 0,6 |
| Tocopherol | 0,5 | 0,5 | 0,5 | 0,5 | 0,5 | 0,5 | 0,5 | 0,5 | 0,5 | 0,5 | 0,5 |
| Tocopheryl Acetate | 0,5 | 0,5 | 0,5 | 0,5 | 0,5 | 0,5 | 0,5 | 0,5 | 0,5 | 0,5 | 0,5 |
| 3,4-Dihydroxy-phenyl-propionsäure- phenethylester | 0,5 | 0,5 | 0,5 | 0,5 | 0,5 | 0,5 | 0,5 | 0,5 | 0,5 | 0,5 | 0,5 |
| Propylparabene | 0,05 | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 | 0,15 |
| Water | ad 100 | | | | | | | | | | |

Tabelle 2: O/W-Emulsionen, Zahlen in Gew.-%

| | 2-1 | 2-2 | 2-3 | 2-4 | 2-5 | 2-6 | 2-7 | 2-8 | 2-9 | 2-10 |
|---|---|---|---|---|---|---|---|---|---|---|
| Titanium dioxide | | 2 | 5 | | | | | | | 3 |
| Methylene Bis-Benztriazolyl Tetramethylbutylphenol | | | | | | 1 | 2 | 1 | | |
| 1-(4-tert-butyl-phenyl)-3-hydroxy-3-(4-methoxy-phenyl)-propan-1-on r | | | | 1 | 2 | | | | 1 | 1 |
| 4-Hydroxy-phenyl-propionsäure-2-ethyl-hexylester | 1 | 3 | | 2 | | 5 | | 5 | 2 | |
| 2-(Hydroxy-phenyl-methyl)-5-methoxy-phenol | 5 | 5 | 5 | 5 | 5 | 5 | 5 | 5 | 5 | 5 |
| 2-[(4-Diethylamino-2-hydroxy-phenyl)-hydroxy-methyl]-benzoesäure-hexylester | 1 | 5 | 4 | | 6 | | 7 | | 2 | 1 |
| 4-Methylbenzyliden Camphor | 2 | | 3 | | 4 | | 3 | | 2 | |
| BMDBM | 1 | 3 | | 3 | 3 | | 3 | 3 | 3 | |
| Stearyl Alcohol (and) Steareth-7 (and) Steareth-10 | 3 | 3 | 3 | 3 | 3 | 3 | 3 | 3 | 3 | 3 |
| Glyceryl Stearate (and) Ceteth-20 | 3 | 3 | 3 | 3 | 3 | 3 | 3 | 3 | 3 | 3 |
| Glyceryl Stearate | 3 | 3 | 3 | 3 | 3 | 3 | 3 | 3 | 3 | 3 |
| Microwax | 1 | 1 | 1 | 1 | 1 | 1 | 1 | 1 | 1 | 1 |
| Cetearyl Octanoate | 11,5 | 11,5 | 11,5 | 11,5 | 11,5 | 11,5 | 11,5 | 11,5 | 11,5 | 11,5 |
| Caprylic/Capric Triglyceride | 6 | 6 | 6 | 6 | 6 | 6 | 6 | 6 | 6 | 6 |
| Oleyl Oleate | 6 | 6 | 6 | 6 | 6 | 6 | 6 | 6 | 6 | 6 |
| Propylene Glycol | 4 | 4 | 4 | 4 | 4 | 4 | 4 | 4 | 4 | 4 |
| Glyceryl Stearate SE | | | | | | | | | | |
| Stearic Acid | | | | | | | | | | |
| Persea Gratissima | | | | | | | | | | |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Tromethamine | | | 1,8 | | | | | | | |
| Water | ad 100 | ad 100 | ad 100 | ad 100 | ad 100 | ad 100 | ad 100 | ad 100 | ad 100 | ad 100 |

Tabelle 2 (Fortsetzung)

| | 2-11 | 2-12 | 2-13 | 2-14 | 2-15 | 2-16 | 2-17 | 2-18 |
|---|---|---|---|---|---|---|---|---|
| Titanium dioxide | 3 | | 2 | | | | 2 | 5 |
| Benzylidene malonate polysiloxane | | 1 | 0,5 | | | | | |
| 2-[(4-Diethylamino-2-hydroxy-phenyl)-hydroxy-methyl]-benzoesäure-hexylester | 1 | 1 | 0,5 | | | | | |
| 4-Hydroxy-3,5-dimethoxy-benzyl-malonsäure-di 2-ethyl-hexylester | | | | 1 | 2 | | | |
| 2-Cyano-3,3,-diphenyl--propionsäure -di 2-ethyl-hexylester | 1 | 3 | | 2 | | 5 | | 5 |
| 5,6,7-Trihydroxyflavon | 5 | 5 | 5 | 5 | 5 | 5 | 5 | 5 |
| 3-(4-tert-butyl-phenyl)-3-hydroxy-1-(4-methoxy-phenyl)-propan-1-on | 1 | 5 | 4 | 1 | 6 | | 7 | |
| Zinc oxide | | | 2 | | | | | |
| UV-Pearl , OMC | 15 | 15 | 15 | 30 | 30 | 30 | 15 | 15 |
| 4-Methylbenzyliden Camphor | | | | 3 | | | | |
| BMDBM | | | | 1 | | | | |
| Phenylbenzimidazole Sulfonic Acid | | | | | 4 | | | |
| Stearyl Alcohol (and) Steareth-7 (and) Steareth-10 | 3 | 3 | 3 | 3 | | | | |
| Glyceryl Stearate (and) Ceteth-20 | 3 | 3 | 3 | 3 | | | | |
| Glyceryl Stearate | 3 | 3 | 3 | 3 | | | | |
| Microwax | 1 | 1 | 1 | 1 | | | | |
| Cetearyl Octanoate | 11,5 | 11,5 | 11,5 | 11,5 | | | | |
| Caprylic/Capric Triglyceride | 6 | 6 | 6 | 6 | 14 | 14 | 14 | 14 |
| Oleyl Oleate | 6 | 6 | 6 | 6 | | | | |
| Propylene Glycol | 4 | 4 | 4 | 4 | | | | |
| Glyceryl Stearate SE | | | | | 6 | 6 | 6 | 6 |
| Stearic Acid | | | | | 2 | 2 | 2 | 2 |
| Persea Gratissima | | | | | 8 | 8 | 8 | 8 |
| Propylparabene | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 |
| Tromethamine | | | | | 1,8 | | | |
| Glycerin | | | | | 3 | 3 | 3 | 3 |
| Water | ad 100 | ad 100 | ad 100 | ad 100 | ad 100 | ad 100 | ad 100 | ad 100 |

Tabelle 2 (Fortsetzung)

| | 2-19 | 2-20 | 2-21 | 2-22 | 2-23 | 2-24 | 2-25 | 2-26 | 2-27 | 2-28 |
|---|---|---|---|---|---|---|---|---|---|---|
| Titanium dioxide | | | | | | | 3 | 3 | | 2 |
| Benzylidene malonate polysiloxane | 1 | 2 | | | | 1 | 1 | | 1 | 0,5 |
| 7,8,3',4´-Tetrahydroxyflavon | | | | 1 | 2 | | | | 1 | 1 |
| 1-(4-tert-butyl-phenyl)-3-hydroxy-3-(4-methoxy-phenyl)-propan-1-on | 1 | 3 | | 2 | | 5 | | 5 | 2 | |
| 2-Cyano-3,3,-diphenyl--propionsäure -di 2-ethyl-hexylester | 5 | 5 | 5 | 5 | 5 | 5 | 5 | 5 | 5 | 5 |
| 4-Hydroxy-3,5-dimethoxy-benzyl-malonsäure-di 2-ethyl-hexylester | 1 | 5 | 4 | | 6 | | 7 | | 2 | 1 |
| 3,4-Dihydroxy-phenyl-propionsäure- phenethylester | | | 1 | 2 | 1 | | | 1 | 1 | 0,5 |
| Zinc oxide | | | | | 5 | 2 | | | | 2 |
| UV-Pearl , OMC | 15 | 15 | 15 | 15 | 15 | 15 | 15 | 15 | 15 | 15 |
| Caprylic/Capric Triglyceride | 14 | 14 | 14 | 14 | 14 | 14 | 14 | 14 | 14 | 14 |
| Oleyl Oleate | | | | | | | | | | |
| Propylene Glycol | | | | | | | | | | |
| Glyceryl Stearate SE | 6 | 6 | 6 | 6 | 6 | 6 | 6 | 6 | 6 | 6 |
| Stearic Acid | 2 | 2 | 2 | 2 | 2 | 2 | 2 | 2 | 2 | 2 |
| Persea Gratissima | 8 | 8 | 8 | 8 | 8 | 8 | 8 | 8 | 8 | 8 |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Glyceryl Stearate, Ceteareth-20, Ceteareth-10, Cetearyl Alcohol, Cetyl Palmitate | | | | | | | | | | |
| Ceteareth-30 | | | | | | | | | | |
| Dicaprylyl Ether | | | | | | | | | | |
| Glycerin | 3 | 3 | 3 | 3 | 3 | 3 | 3 | 3 | 3 | 3 |
| Water | ad 100 | ad 100 | ad 100 | ad 100 | ad 100 | ad 100 | ad 100 | ad 100 | ad 100 | ad 100 |

Tabelle 3: Gele, Zahlen in Gew.-%

| | 3-1 | 3-2 | 3-3 | 3-4 | 3-5 | 3-6 | 3-7 | 3-8 | 3-9 | 3-10 |
|---|---|---|---|---|---|---|---|---|---|---|
| A = aqueous gel | | | | | | | | | | |
| Titanium dioxide | | 2 | 5 | | | | | | | 3 |
| 5,6,7-Trihydroxyflavon | | | | 1 | 2 | | | | 1 | 1 |
| 4-Hydroxy-3,5-dimethoxy-benzyl-malonsäure-di 2-ethyl-hexylester | 1 | 3 | | 2 | | 5 | | 5 | 2 | |
| 2-Cyano-3,3,-diphenyl--propionsäure -di 2-ethyl-hexylester | 5 | 5 | 5 | 5 | 5 | 5 | 5 | 5 | 5 | 5 |
| 2-[(4-Diethylamino-2-hydroxy-phenyl)-hydroxy-methyl]-benzoesäure-hexylester | 1 | 5 | 4 | | 6 | | 7 | | 2 | 1 |
| 1-(4-tert-butyl-phenyl)-3-hydroxy-3-(4-methoxy-phenyl)-propan-1-on | | | 1 | 1 | 2 | | | | 1 | 1 |
| Methylene Bis-Benztriazolyl Tetramethylbutylphenol | | 1 | | | | 1 | 2 | 1 | | |
| Zinc oxide | | | | 2 | | | | 5 | 2 | |
| UV-Pearl , Ethylhexyl Mehtoxycinnamat | 30 | 15 | 15 | 15 | 15 | 15 | 15 | 15 | 15 | 15 |
| 4-Methylbenzyliden Camphor | | | | | 2 | | | | | |
| Butylmethoxydibenzoylmethane | | 1 | | | | | | | | |
| Phenylbenzimidazole Sulfonic Acid | | | 4 | | | | | | | |
| Prunus Dulcis | 5 | 5 | 5 | 5 | 5 | 5 | 5 | 5 | 5 | 5 |
| Tocopheryl Acetate | 0,5 | 0,5 | 0,5 | 0,5 | 0,5 | 0,5 | 0,5 | 0,5 | 0,5 | 0,5 |
| Caprylic/Capric Triglyceride | 3 | 3 | 3 | 3 | 3 | 3 | 3 | 3 | 3 | 3 |
| Octyldodecanol | 2 | 2 | 2 | 2 | 2 | 2 | 2 | 2 | 2 | 2 |
| Decyl Oleate | 2 | 2 | 2 | 2 | 2 | 2 | 2 | 2 | 2 | 2 |
| PEG-8 (and) Tocopherol (and) Ascorbyl Palmitate (and) Ascorbic Acid (and) Citric Acid | 0,05 | 0,05 | 0,05 | 0,05 | 0,05 | 0,05 | 0,05 | 0,05 | 0,05 | 0,05 |
| Sorbitol | 4 | 4 | 4 | 4 | 4 | 4 | 4 | 4 | 4 | 4 |
| Polyacrylamide (and) C13-14 Isoparaffin (and) Laureth-7 | 3 | 3 | 3 | 3 | 3 | 3 | 3 | 3 | 3 | 3 |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Tromethamine | | | 1,8 | | | | | | | |
| Water | ad 100 | ad 100 | ad 100 | ad 100 | ad 100 | ad 100 | ad 100 | ad 100 | ad 100 | ad 100 |

Tabelle 3 (Fortsetzung)

| | 3-11 | 3-12 | 3-13 | 3-14 | 3-15 | 3-16 | 3-17 | 3-18 |
|---|---|---|---|---|---|---|---|---|
| a = aqueaous gel | | | | A | a | a | a | a |
| Titanium dioxide | 3 | | 2 | | | | | |
| Benzylidene malonate polysiloxane | | 1 | 0,5 | 1 | 2 | | | |
| Methylene Bis-Benztriazolyl Tetramethylbutylphenol | 1 | 1 | 0,5 | | | 1 | 2 | 1 |
| 4-Hydroxy-3,5-dimethoxy-benzyl-malonsäure-di 2-ethyl-hexylester | | | | 1 | 2 | | | |
| 2-(Hydroxy-phenyl-methyl)-5-methoxy-phenol | 1 | 3 | | 2 | | 5 | | 5 |
| 2-Cyano-3,3,-diphenyl--propionsäure -di 2-ethyl-hexylester | 5 | 5 | 5 | 5 | 5 | 5 | 5 | 5 |
| 6,3',4'-Trihydroxyflavon | 1 | 5 | 4 | | 6 | | 7 | |
| Zinc oxide | | | 2 | | | | | |
| UV-Pearl , Ethylhexyl Mehtoxycinnamat | 15 | 15 | 15 | 15 | 15 | 15 | 15 | 15 |
| Prunus Dulcis | 5 | 5 | 5 | | | | | |
| Tocopheryl Acetate | 0,5 | 0,5 | 0,5 | | | | | |
| Caprylic/Capric Triglyceride | 3 | 3 | 3 | | | | | |
| Octyldodecanol | 2 | 2 | 2 | | | | | |
| Decyl Oleate | 2 | 2 | 2 | | | | | |
| PEG-8 (and) Tocopherol (and) Ascorbyl Palmitate (and) Ascorbic Acid (and) Citric Acid | 0,05 | 0,05 | 0,05 | | | | | |
| Sorbitol | 4 | 4 | 4 | 5 | 5 | 5 | 5 | 5 |
| Polyacrylamide (and) C13-14 Isoparaffin (and) Laureth-7 | 3 | 3 | 3 | | | | | |
| Carbomer | | | | 1,5 | 1,5 | 1,5 | 1,5 | 1,5 |
| Propylparabene | 0,05 | 0,05 | 0,05 | | | | | |
| Methylparabene | 0,15 | 0,15 | 0,15 | 0,15 | 0,15 | 0,15 | 0,15 | 0,15 |
| Allantoin | | | | 0,2 | 0,2 | 0,2 | 0,2 | 0,2 |
| Tromethamine | | | | 2,4 | 2,4 | 2,4 | 2,4 | 2,4 |
| Water | ad 100 | ad 100 | ad 100 | ad 100 | ad 100 | ad 100 | ad 100 | ad 100 |

Tabelle 3 (Fortsetzung)

| | 3-19 | 3-20 | 3-21 | 3-22 | 3-23 | 3-24 | 3-25 | 3-26 | 3-27 | 3-28 |
|---|---|---|---|---|---|---|---|---|---|---|
| 7,8,3',4'-Tetrahydroxyflavon | | | | 1 | 2 | | | | 1 | 1 |
| 2-[(4-Diethylamino-2-hydroxy-phenyl)-hydroxy-methyl]-benzoesäure-hexylester | 1 | 3 | | 2 | | 5 | | 5 | 2 | |
| 2-Cyano-3,3,-diphenyl--propionsäure -di 2-ethyl-hexylester | 5 | 5 | 5 | 5 | 5 | 5 | 5 | 5 | 5 | 5 |
| 1-(4-tert-butyl-phenyl)-3-hydroxy-3-(4-methoxy-phenyl)-propan-1-on | 1 | 5 | 4 | | 6 | | 7 | | 2 | 1 |
| UV-Pearl , OMC | 30 | 30 | 15 | 15 | 15 | 11 | 12 | 15 | 15 | 15 |
| Phenylbenzimidazole Sulfonic Acid | | 4 | 4 | | | | | | | |
| Sorbitol | 5 | 5 | 5 | 5 | 5 | 5 | 5 | 5 | 5 | 5 |
| Carbomer | 1,5 | 1,5 | 1,5 | 1,5 | 1,5 | 1,5 | 1,5 | 1,5 | 1,5 | 1,5 |
| Propylparabene | | | | | | | | | | |
| Methylparabene | 0,15 | 0,15 | 0,15 | 0,15 | 0,15 | 0,15 | 0,15 | 0,15 | 0,15 | 0,15 |
| Allantoin | 0,2 | 0,2 | 0,2 | 0,2 | 0,2 | 0,2 | 0,2 | 0,2 | 0,2 | 0,2 |
| Tromethamine | 2,4 | 4,2 | 4,2 | 2,4 | 2,4 | 2,4 | 2,4 | 2,4 | 2,4 | 2,4 |
| Water | ad 100 | ad 100 | ad 100 | ad 100 | ad 100 | ad 100 | Ad 100 | ad 100 | ad 100 | ad 100 |

Tabelle 3 (Fortsetzung)

| | 3-29 | 3-30 | 3-31 | 3-32 | 3-33 | 3-34 | 3-35 | 3-36 |
|---|---|---|---|---|---|---|---|---|
| 1-(4-tert-butyl-phenyl)-3-hydroxy-3-(4-methoxy-phenyl)-propan-1-on | | | | 1 | 2 | | | |
| 2-Cyano-3,3,-diphenyl--propionsäure -di 2-ethyl-hexylester | 1 | 3 | | 2 | | 5 | | 5 |
| 2-[(4-Diethylamino-2-hydroxy-phenyl)-hydroxy-methyl]-benzoesäure-hexylester | 5 | 5 | 5 | 5 | 5 | 5 | 5 | 5 |
| 5,6,7-Trihydroxyflavon | 1 | 5 | 4 | | 6 | | 7 | |
| UV-Pearl , OMC | 15 | 10 | | 10 | 10 | 10 | 15 | 10 |
| UV-Pearl , OCR | | | 10 | | | | | |
| UV-Pearl , OMC, Methylene Bis-Benztriazolyl Tetramethylbutylphenol | | 7 | | 6 | | | | |
| UV-Pearl, Ethylhexyl salicylate, BMDBM | | | 10 | | | | | |
| Disodium Phenyl Dibenzimidazole Tetrasulfonate | | 3 | | | | 3 | | 3 |
| Phenylbenzimidazole Sulfonic Acid | | 2 | | | 2 | 3 | | 3 |
| Prunus Dulcis | 5 | 5 | 5 | | | | | |
| Tocopheryl Acetate | 0,5 | 0,5 | 0,5 | | | | | |
| Caprylic/Capric Triglyceride | 3 | 3 | 3 | | | | | |
| Octyldodecanol | 2 | 2 | 2 | | | | | |
| Decyl Oleate | 2 | 2 | 2 | | | | | |
| PEG-8 (and) Tocopherol (and) Ascorbyl Palmitate (and) Ascorbic Acid (and) Citric Acid | 0,05 | 0,05 | 0,05 | | | | | |
| Sorbitol | 4 | 4 | 4 | 5 | 5 | 5 | 5 | 5 |
| Polyacrylamide (and) C13-14 Isoparaffin (and) Laureth-7 | 3 | 3 | 3 | | | | | |
| Carbomer | | | | 1,5 | 1,5 | 1,5 | 1,5 | 1,5 |
| Propylparabene | 0,05 | 0,05 | 0,05 | | | | | |
| Methylparabene | 0,15 | 0,15 | 0,15 | 0,15 | 0,15 | 0,15 | 0,15 | 0,15 |
| Allantoin | | | | 0,2 | 0,2 | 0,2 | 0,2 | 0,2 |
| Tromethamine | | | | 2,4 | 2,4 | 2,4 | 2,4 | 2,4 |
| Water | ad 100 | ad 100 | ad 100 | ad 100 | ad 100 | ad 100 | ad 100 | ad 100 |

**Patentansprüche**

**1.** Verwendung mindestens einer Verbindung der Formel Ia, Ib, Ic oder Id

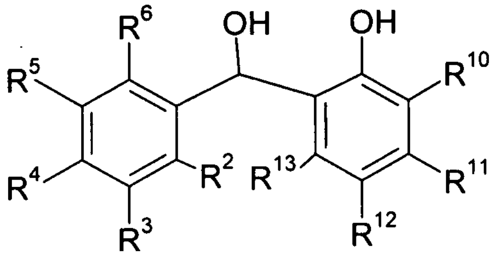

Ia

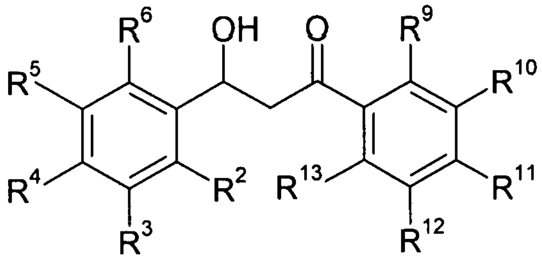

Ib

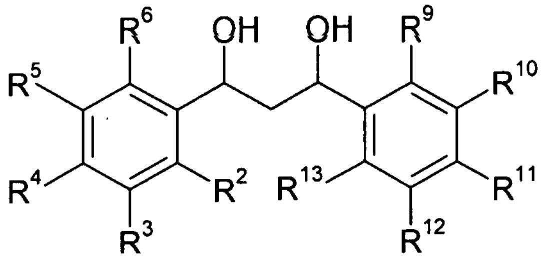

Ic

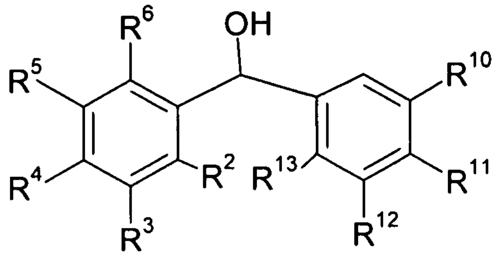

Id

wobei

$R^2$ bis $R^6$ und $R^9$ bis $R^{13}$ jeweils unabhängig voneinander ausgewählt sind aus H,
OH,
geradkettigen oder verzweigten $C_1$- bis $C_{20}$-Alkoxygruppen, wobei die Alkylketten jeweils auch durch Sauerstoff oder Stickstoff unterbrochen sein können,
geradkettigen oder verzweigten $C_1$- bis $_{20}$-Alkylgruppen, wobei die Alkylketten jeweils auch durch Sauerstoff oder Stickstoff unterbrochen sein können,
geradkettigen oder verzweigten $C_3$- bis $C_{20}$-Alkenylgruppen,
geradkettigen oder verzweigten $C_1$- bis $C_{20}$-Hydroxyalkylgruppen, wobei die Hydroxygruppe an ein primäres oder sekundäres Kohlenstoffatom der Kette gebunden sein kann und weiter die Alkylketten jeweils auch durch Sauerstoff oder Stickstoff unterbrochen sein können,
geradkettigen oder verzweigten $C_1$- bis $C_{20}$-Hydroxyalkoxygruppen, wobei die Hydroxygruppe(n) an primäre oder sekundäre Kohlenstoffatome der Kette gebunden sein können und weiter die Alkylkette auch durch Sauerstoff unterbrochen sein kann,
geradkettigen oder verzweigten $C_1$- bis $C_{20}$-Alkylaminogruppen,
geradkettigen oder verzweigten $C_1$- bis $C_{20}$-Dialkylaminogruppen,
oder $R^2$ bis $R^6$ und $R^9$ bis $R^{13}$ stehen jeweils unabhängig voneinander für eine Carbonsäure-, Phosphorsäure-,

Sulfonsäure-, Schwefelsäure- oder Sulfonfunktion, die optional mit geradkettigen oder verzweigten $C_1$- bis $_{20}$-Alkylgruppen bzw. geradkettigen oder verzweigten $C_3$- bis $C_{20}$-Alkenylgruppen verestert oder alkyliert sein kann,
oder es sich um Salze der Verbindungen der Formel la bis Id handelt als Antioxidans zur Herstellung kosmetischer oder pharmazeutischer, insbesondere dermatologischer Zubereitungen oder von Nahrungsmitteln bzw. Nahrungsergänzungsmitteln oder zur Herstellung von Haushaltsprodukten.

2. Verwendung nach Anspruch 1, **dadurch gekennzeichnet dass**
$R^4$ und $R^{11}$ jeweils unabhängig voneinander ausgewählt sind aus H, geradkettigen oder verzweigten $C_1$- bis $C_{20}$-Alkoxygruppen, geradkettigen oder verzweigten $C_1$- bis $C_{20}$-Alkylgruppen oder geradkettigen oder verzweigten $C_1$- bis $C_{20}$-Dialkylaminogruppen.

3. Verwendung nach Anspruch 1 oder 2, **dadurch gekennzeichnet, dass**
$R^2$, $R^6$ und $R^{13}$ jeweils H bedeuten.

4. Verwendung nach einem oder mehreren der Ansprüche 1 bis 3, **dadurch gekennzeichnet, dass** $R^3$, $R^5$, $R^9$, $R^{10}$ und $R^{12}$ H bedeuten.

5. Verwendung nach einem oder mehreren der Ansprüche 1 bis 4, **dadurch gekennzeichnet, dass** $R^3$ oder $R^{12}$ $SO_3H$ oder Sulfonat bedeutet.

6. Verwendung nach einem oder mehreren der Ansprüche 1 bis 5, **dadurch gekennzeichnet dass**
$R^4$ ausgewählt wird aus H, $C_1$- bis $C_4$-Alkoxygruppen oder $C_1$- bis $C_4$-Dialkylaminogruppen,
$R^{11}$ ausgewählt wird aus geradkettigen oder verzweigten $C_1$- bis $C_6$-Alkylgruppen,
$C_1$- bis $C_4$-Dialkylaminogruppen
$C_1$- bis $C_4$-Alkoxygruppen.

7. Verwendung nach einem oder mehreren der Ansprüche 1 bis 6, **dadurch gekennzeichnet, dass** $R^6$ $C_1$- bis $C_{10}$-Alkoxycarbonyl bedeutet.

8. Verwendung mindestens einer Verbindung der Formel Ia, Ib, Ic oder Id nach einem oder mehreren der Ansprüche 1 bis 7, **dadurch gekennzeichnet dass**
die mindestens eine Verbindung ausgewählt ist aus 2-(Hydroxy-phenyl-methyl)-5-diethylamino-phenol, 2-(Hydroxy-phenyl-methyl)-phenol, 2-(Hydroxy-phenyl-methyl)-5-sulfo-phenol, 2-(Hydroxy-phenyl-methyl)-5-methoxy-4-sulfo-phenol, 2-(Hydroxy-phenyl-methyl)-5-methoxy-phenol, 2-[(4-Diethylamino-2-hydroxy-phenyl)-hydroxy-methyl]-benzoesäure-ethylester, 2-[(4-Diethylamino-2-hydroxy-phenyl)-hydroxy-methyl]-benzoesäure-hexylester, 2-[(4-Diethylamino-2-hydroxy-phenyl)-hydroxy-methyl]-benzoesäure-ethylhexylester, 2-[(2-hydroxy-phenyl)-hydroxy-methyl]-benzoesäure-ethylhexylester, 3-Hydroxy-1,3-diphenyl-propan-1-on, 1-(4-tert-butyl-phenyl)-3-hydroxy-3-phenyl-propan-1-on, 1-(4-tert-butyl-phenyl)-3-hydroxy-3-(4-methoxy-phenyl)-propan-1-on, 3-(4-tert-butyl-phenyl)-3-hydroxy-1-phenyl-propan-1-on, 3-(4-tert-butyl-phenyl)-3-hydroxy-1-(4-methoxy-phenyl)-propan-1-on oder 3-(4-methoxy-phenyl)-3-hydroxy-1-(4-methoxy-phenyl)-propan-1-on.

9. Verbindungen der Formel Ib oder Ic

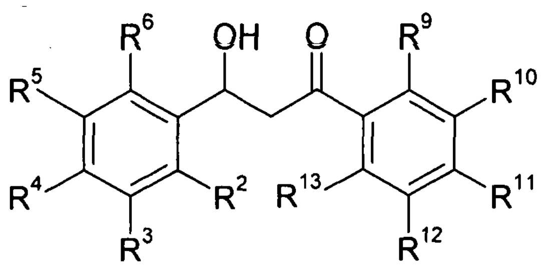

Ib

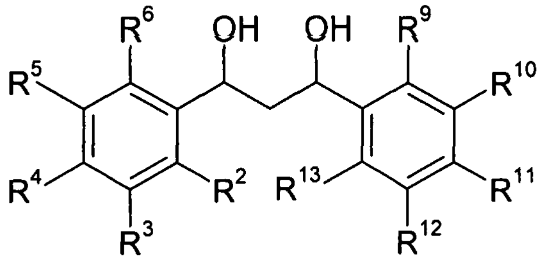

Ic

wobei

$R^4$ und $R^{11}$ jeweils unabhängig voneinander H, geradkettige oder verzweigte $C_1$- bis $C_{20}$-Alkylgruppe, geradkettige oder verzweigte $C_1$-bis $C_{20}$-Alkoxygruppe oder geradkettige oder verzweigte $C_1$- bis $C_{20}$-Dialkylaminogruppe,
$R^6$ H oder eine Carbonsäure-, Phosphorsäure-, Sulfonsäure-, Schwefelsäure- oder Sulfonfunktion, die mit geradkettigen oder verzweigten $C_1$- bis $C_{20}$-Alkylgruppen oder geradkettigen oder verzweigten $C_3$- bis $C_{20}$-Alkenylgruppen verestert oder alkyliert sein kann und
$R^2$, $R^3$, $R^5$, $R^9$, $R^{10}$, $R^{12}$ und $R^{13}$ H bedeuten.

**10.** Verbindungen nach Anspruch 9, ausgewählt aus der Gruppe

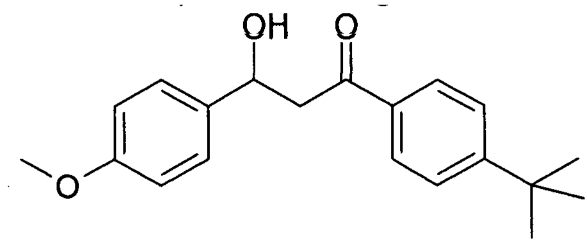

(1)

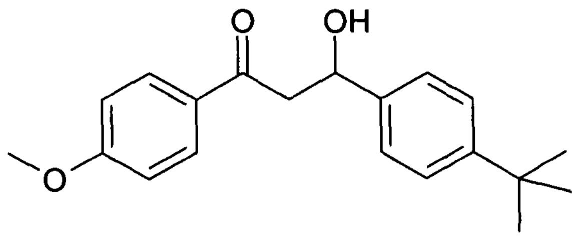

(2)

oder

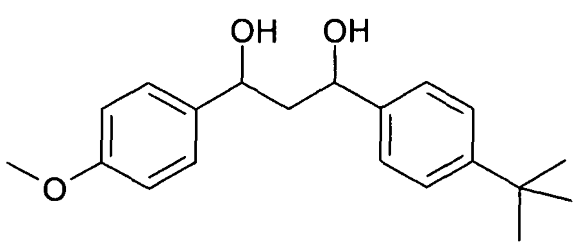

(3)

oder Salze der Verbindungen.

**11.** Verbindungen nach Formel Ia ausgewählt aus

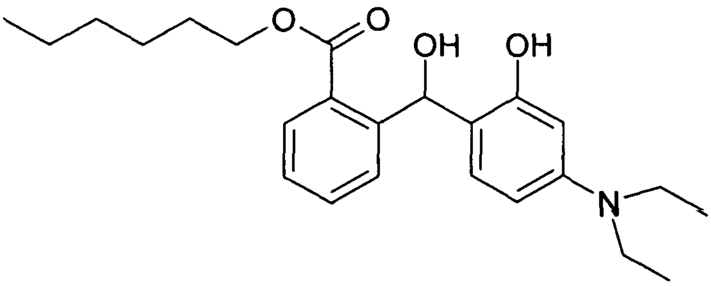

(4)

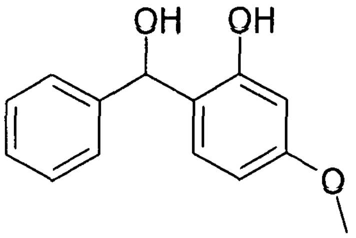

(5)

oder

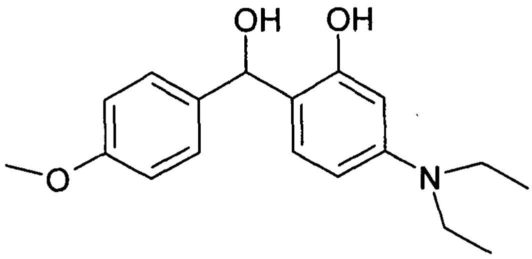

(6)

oder Salze der Verbindungen.

**12.** Zubereitung enthaltend mindestens einen für kosmetische oder pharmazeutische, insbesondere dermatologische Zubereitungen, Nahrungsmittel bzw. Nahrungsergänzungsmittel oder Haushaltsprodukte geeigneten Träger und zumindest eine Verbindung der Formeln Ia bis Id mit Resten gemäß mindestens einem der Ansprüche 1 bis 11.

**13.** Zubereitung nach Anspruch 12, **dadurch gekennzeichnet, dass** die Zubereitungen die eine oder mehreren Verbindungen nach Formeln Ia bis Id in einer Menge von 0,01 bis 20 Gew.-% enthalten.

**14.** Zubereitung nach einem oder mehreren der Ansprüche 12 und 13 zum Schutz von Körperzellen gegen oxidativen Stress, **dadurch gekennzeichnet, dass** sie ein oder mehrere weitere Antioxidantien und/oder Vitamine enthält.

**15.** Zubereitung nach einem oder mehreren der Ansprüche 12 bis 14, **dadurch gekennzeichnet, dass** die Zubereitung mindestens einen Selbstbräuner enthält.

**16.** Verfahren zur Herstellung einer Zubereitung nach Anspruch 12, **dadurch gekennzeichnet, dass** eine Verbindung nach Formeln Ia bis Id mit Resten nach einem oder mehreren der Ansprüche 1 bis 10 mit einem kosmetisch oder pharmzeutisch oder für Nahrungsmittel oder Nahrungsergänzungsmittel oder für Haushaltsprodukte geeignetem Träger vermischt wird.

**17.** Verfahren zur Herstellung einer Verbindung der Formel Ib oder Ic

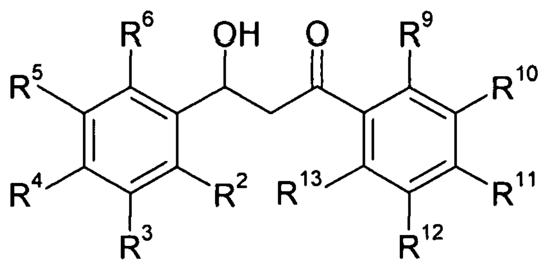

Ib

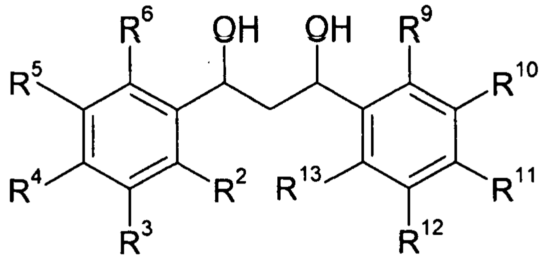

Ic

wobei

$R^4$ und $R^{11}$ jeweils unabhängig voneinander H, geradkettige oder verzweigte $C_1$- bis $C_{20}$-Alkylgruppe, geradkettige oder verzweigte $C_1$- bis $C_{20}$-Alkoxygruppe oder geradkettige oder verzweigte $C_1$- bis $C_{20}$-Dialkylaminogruppe,
$R^6$ H oder eine Carbonsäure-, Phosphorsäure-, Sulfonsäure-, Schwefelsäure- oder Sulfonfunktion, die mit geradkettigen oder verzweigten $C_1$- bis $C_{20}$-Alkylgruppen oder geradkettigen oder verzweigten $C_3$- bis $C_{20}$-Alkenylgruppen verestert oder alkyliert sein kann und
$R^2$, $R^3$, $R^5$, $R^9$, $R^{10}$, $R^{12}$ und $R^{13}$ H bedeuten, **dadurch gekennzeichnet, dass** eine Verbindung der Formel Ib en

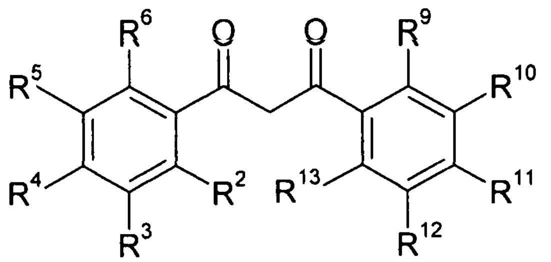

Ib en

wobei die Reste $R^2$ bis $R^6$ und $R^9$ bis $R^{13}$ eine zuvor genannte Bedeutung haben, hydriert wird.

**18.** Verwendung von Verbindungen der Formel Ia, Ib, Ic oder Id gemäß Anspruch 1 zum Schutz oxidationsempfindlicher Formulierungsbestandteile wie organischer oder anorganischer Farbstoffe, Antioxidantien, Vitamine, Parfumkomponenten, Ölkomponenten oder Matrixbestandteile, wie Emulgatoren, Verdicker, Filmbildner und Tenside.

**19.** Verbindungen der Formel Ia, Ib, Ic oder Id gemäß Anspruch 1 zur Verwendung bei der Pigmentierungskontrolle, insbesondere zur Aufhellung von Hautpartien.

**Claims**

**1.** Use of at least one compound of the formula Ia, Ib, Ic or Id

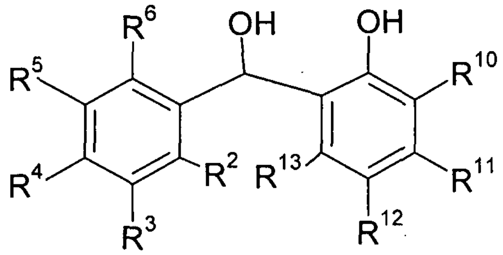

Ia

,

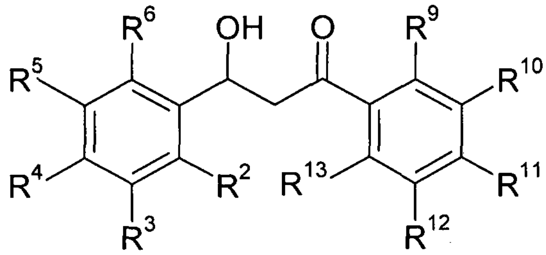

Ib

,

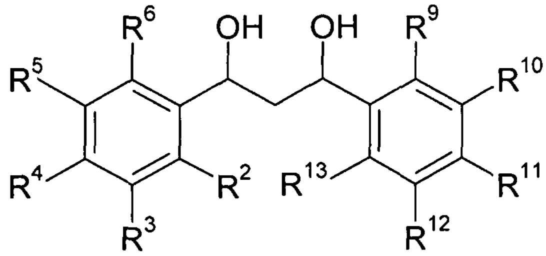

Ic

,

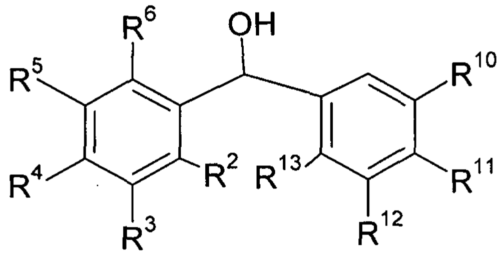

Id

,

where

$R^2$ to $R^6$ and $R^9$ to $R^{13}$ are each selected, independently of one another, from H,
OH,
straight-chain or branched $C_1$- to $C_{20}$-alkoxy groups, where the alkyl chains may each also be interrupted by oxygen or nitrogen, straight-chain or branched $C_1$- to $C_{20}$-alkyl groups, where the alkyl chains may each also be interrupted by oxygen or nitrogen, straight-chain or branched $C_3$- to $C_{20}$-alkenyl groups,
straight-chain or branched $C_1$- to $C_{20}$-hydroxyalkyl groups, where the hydroxyl group may be bonded to a primary or secondary carbon atom of the chain and furthermore the alkyl chains may each also be interrupted by oxygen or nitrogen,
straight-chain or branched $C_1$- to $C_{20}$-hydroxyalkoxy groups, where the hydroxyl group(s) may be bonded to primary or secondary carbon atoms of the chain and furthermore the alkyl chain may also be interrupted by oxygen,
straight-chain or branched $C_1$- to $C_{20}$-alkylamino groups,
straight-chain or branched $C_1$- to $C_{20}$-dialkylamino groups,
or $R^2$ to $R^6$ and $R^9$ to $R^{13}$ each stand, independently of one another, for a carboxylic acid, phosphoric acid,

sulfonic acid, sulfuric acid or sulfone function, which may optionally be esterified or alkylated by means of straight-chain or branched $C_1$- to $C_{20}$-alkyl groups or straight-chain or branched $C_3$- to $C_{20}$-alkenyl groups, or salts of the compounds of the formulae Ia to Id,
as antioxidant for the preparation of cosmetic or pharmaceutical, in particular dermatological compositions or of foods or food supplements or for the preparation of household products.

2. Use according to Claim 1, **characterised in that** $R^4$ and $R^{11}$ are each selected, independently of one another, from H, straight-chain or branched $C_1$- to $C_{20}$-alkoxy groups, straight-chain or branched $C_1$- to $C_{20}$-alkyl groups or straight-chain or branched $C_1$- to $C_{20}$-dialkylamino groups.

3. Use according to Claim 1 or 2, **characterised in that** $R^2$, $R^6$ and $R^{13}$ each denote H.

4. Use according to one or more of Claims 1 to 3, **characterised in that** $R^3$, $R^5$, $R^9$, $R^{10}$ and $R^{12}$ denote H.

5. Use according to one or more of Claims 1 to 4, **characterised in that** $R^3$ or $R^{12}$ denotes $SO_3H$ or sulfonate.

6. Use according to one or more of Claims 1 to 5, **characterised in that**
$R^4$ is selected from H, $C_1$- to $C_4$-alkoxy groups or $C_1$- to $C_4$-dialkylamino groups,
$R^{11}$ is selected from straight-chain or branched $C_1$- to $C_6$-alkyl groups, $C_1$- to $C_4$-dialkylamino groups, $C_1$- to $C_4$-alkoxy groups.

7. Use according to one or more of Claims 1 to 6, **characterised in that** $R^6$ denotes $C_1$- to $C_{10}$-alkoxycarbonyl.

8. Use of at least one compound of the formula Ia, Ib, Ic or Id according to one or more of Claims 1 to 7, **characterised in that** the at least one compound is selected from 2-(hydroxyphenylmethyl)-5-diethylamino-phenol, 2-(hydroxyphenylmethyl)phenol, 2-(hydroxyphenylmethyl)-5-sulfophenol, 2-(hydroxyphenylmethyl)-5-methoxy-4-sulfophenol, 2-(hydroxyphenylmethyl)-5-methoxyphenol, ethyl 2-[(4-diethylamino-2-hydroxyphenyl)hydroxymethyl]benzoate, hexyl 2-[(4-diethylamino-2-hydroxyphenyl)hydroxymethyl]benzoate, ethylhexyl 2-[(4-diethylamino-2-hydroxyphenyl)hydroxymethyl]benzoate, ethylhexyl 2-[(2-hydroxyphenyl)hydroxymethyl]benzoate, 3-hydroxy-1,3-diphenylpropan-1-one, 1-(4-tert-butylphenyl)-3-hydroxy-3-phenylpropan-1-one, 1-(4-tert-butylphenyl)-3-hyd roxy-3-(4-methoxyphenyl)propan-1-one, 3-(4-tert-butylphenyl)-3-hydroxy-1-phenylpropan-1-one, 3-(4-tert-butylphenyl)-3-hydroxy-1-(4-methoxyphenyl)propan-1-one or 3-(4-methoxyphenyl)-3-hydroxy-1-(4-methoxyphenyl)propan-1-one.

9. Compounds of the formula Ib or Ic

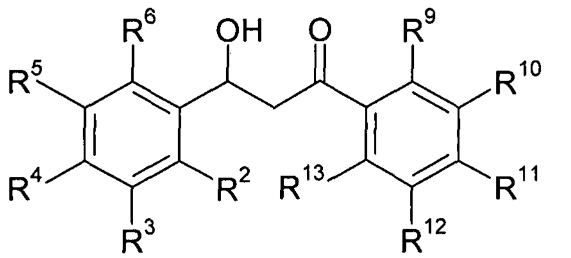

Ib

,

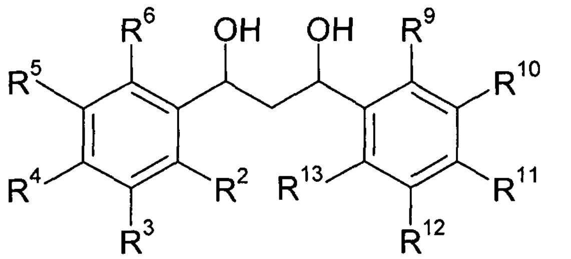

Ic

,

where

$R^4$ and $R^{11}$ each, independently of one another, denote H, a straight-chain or branched $C_1$- to $C_{20}$-alkyl group, a straight-chain or branched $C_1$- to $C_{20}$-alkoxy group or a straight-chain or branched $C_1$- to $C_{20}$-dialkylamino group,
$R^6$ denotes H or a carboxylic acid, phosphoric acid, sulfonic acid, sulfuric acid or sulfone function, which may be esterified or alkylated by means of straight-chain or branched $C_1$- to $C_{20}$-alkyl groups or straight-chain or branched $C_3$- to $C_{20}$-alkenyl groups, and
$R^2$, $R^3$, $R^5$, $R^9$, $R^{10}$, $R^{12}$ and $R^{13}$ denote H.

**10.** Compounds according to Claim 9, selected from the group

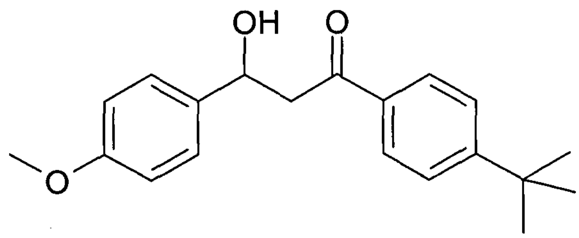

(1)

,

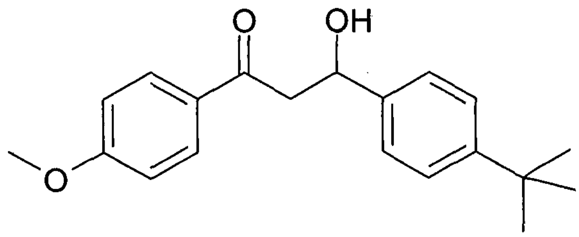

(2)

or

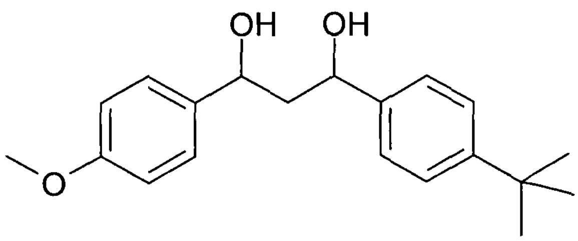

(3)

or salts of the compounds.

**11.** Compounds of the formula Ia, selected from

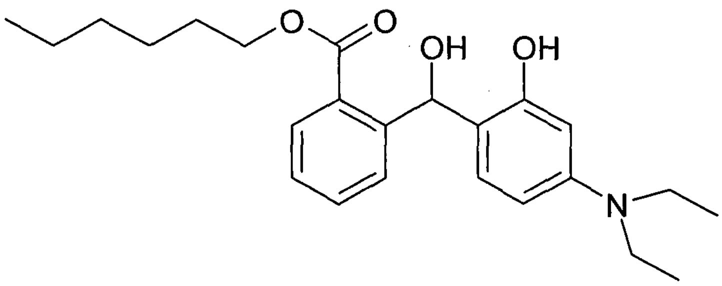

(4)

,

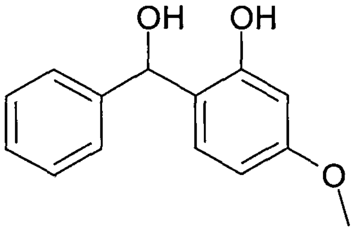

(5)

or

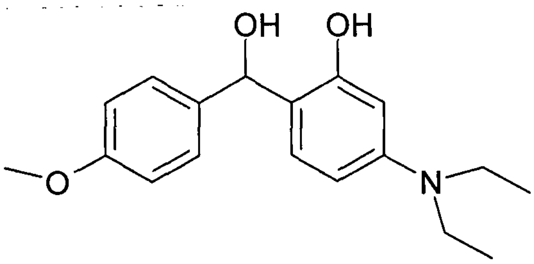

(6)

or salts of the compounds.

**12.** Composition comprising at least one vehicle which is suitable for cosmetic or pharmaceutical, in particular dermatological compositions, foods or food supplements or household products and at least one compound of the formulae Ia to Id containing radicals according to at least one of Claims 1 to 11.

**13.** Composition according to Claim 12, **characterised in that** the compositions comprise the one or more compounds of the formulae Ia to Id in an amount of 0.01 to 20% by weight.

**14.** Composition according to one or more of Claims 12 and 13 for the protection of body cells against oxidative stress, **characterised in that** it comprises one or more further antioxidants and/or vitamins.

**15.** Composition according to one or more of Claims 12 to 14, **characterised in that** the composition comprises at least one self-tanning agent.

**16.** Process for the preparation of a composition according to Claim 12, **characterised in that** a compound of the formulae Ia to Id containing radicals according to one or more of Claims 1 to 10 is mixed with a vehicle which is suitable cosmetically or pharmaceutically or for foods or food supplements or for household products.

**17.** Process for the preparation of a compound of the formula Ib or Ic

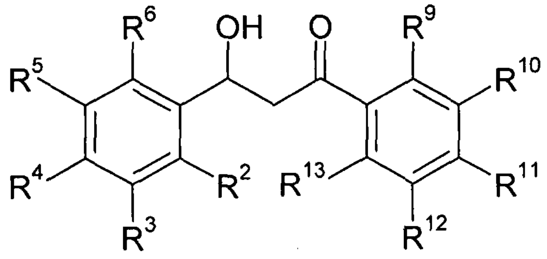

Ib

,

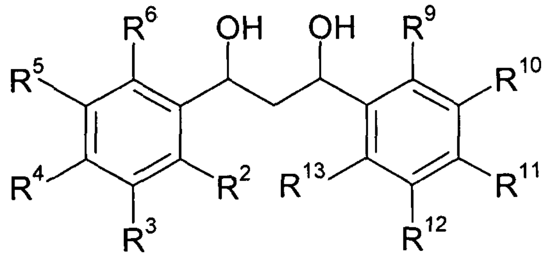

Ic

,

where

$R^4$ and $R^{11}$ each, independently of one another, denote H, a straight-chain or branched $C_1$- to $C_{20}$-alkyl group, a straight-chain or branched $C_1$- to $C_{20}$-alkoxy group or a straight-chain or branched $C_1$- to $C_{20}$-dialkylamino group,
$R^6$ denotes H or a carboxylic acid, phosphoric acid, sulfonic acid, sulfuric acid or sulfone function, which may be esterified or alkylated by means of straight-chain or branched $C_1$- to $C_{20}$-alkyl groups or straight-chain or branched $C_3$- to $C_{20}$-alkenyl groups, and
$R^2$, $R^3$, $R^5$, $R^9$, $R^{10}$, $R^{12}$ and $R^{13}$ denote H, **characterised in that** a compound of the formula Ib en

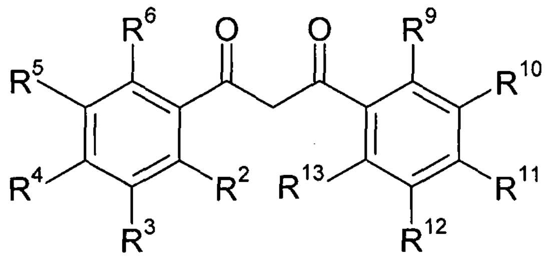

Ib en

,

where the radicals $R^2$ to $R^6$ and $R^9$ to $R^{13}$ have a meaning given above, is hydrogenated.

**18.** Use of compounds of the formula Ia, Ib, Ic or Id according to Claim 1 for the protection of oxidation-sensitive formulation constituents, such as organic or inorganic dyes, antioxidants, vitamins, perfume components, oil components or matrix constituents, such as emulsifiers, thickeners, film formers and surfactants.

**19.** Compounds of the formula Ia, Ib, Ic or Id according to Claim 1 for use in pigmentation control, in particular for lightening skin areas.

**Revendications**

**1.** Utilisation d'au moins un composé de formule Ia, Ib, Ic ou Id

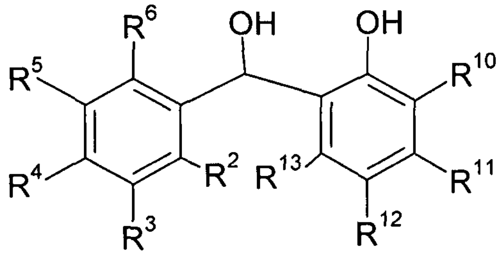

Ia

,

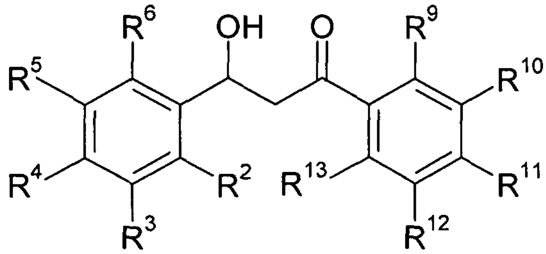

Ib

,

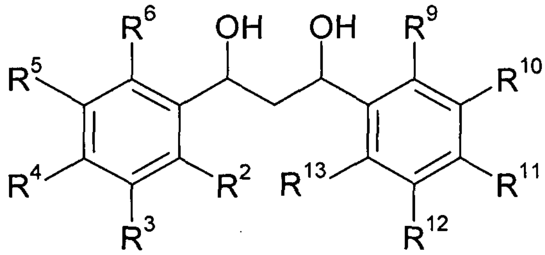

Ic

,

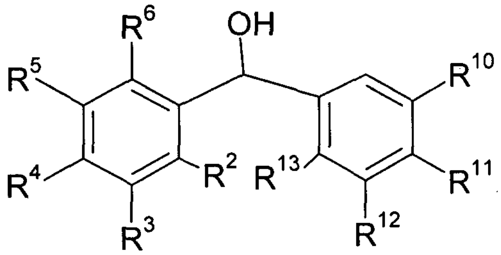

Id

,

dans laquelle

$R^2$ à $R^6$ et $R^9$ à $R^{13}$ sont choisis chacun, indépendamment les uns des autres, parmi H,
OH,
des groupements $C_1$- à $C_{20}$-alcoxy à chaîne linéaire ou ramifiée, où les chaînes alkyle peuvent chacune être également interrompues par oxygène ou azote,
des groupements $C_1$- à $C_{20}$-alkyle à chaîne linéaire ou ramifiée, où les chaînes alkyle peuvent chacune être également interrompues par oxygène ou azote,
des groupements $C_3$- à $C_{20}$-alcényle à chaîne linéaire ou ramifiée, des groupements $C_1$- à $C_{20}$-hydroxyalkyle à chaîne linéaire ou ramifiée, où le groupement hydroxyle peut être lié à un atome de carbone primaire ou secondaire de la chaîne et en outre les chaînes alkyle peuvent chacune être également interrompues par oxygène ou azote,
des groupements $C_1$- à $C_{20}$-hydroxyalcoxy à chaîne linéaire ou ramifiée, où le ou les groupements hydroxyle peuvent être liés à des atomes de carbone primaires ou secondaires de la chaîne et en outre la chaîne alkyle peut être également interrompue par oxygène,
des groupements $C_1$- à $C_{20}$-alkylamino à chaîne linéaire ou ramifiée, des groupements $C_1$- à $C_{20}$-dialkylamino à chaîne linéaire ou ramifiée,

ou $R^2$ à $R^6$ et $R^9$ à $R^{13}$ représentent chacun, indépendamment les uns des autres, une fonction acide carboxylique, acide phosphorique, acide sulfonique, acide sulfurique ou sulfone, pouvant éventuellement être estérifiée ou alkylée au moyen de groupements $C_1$- à $C_{20}$-alkyle à chaîne linéaire ou ramifiée ou de groupements $C_3$- à $C_{20}$-alcényle à chaîne linéaire ou ramifiée,
ou des sels des composés de formules la à Id,
comme antioxydant pour la préparation de compositions cosmétiques ou pharmaceutiques, en particulier dermatologiques ou d'aliments ou de compléments alimentaires ou pour la préparation de produits ménagers.

2. Utilisation selon la revendication 1, **caractérisée en ce que** $R^4$ et $R^{11}$ sont choisis chacun, indépendamment l'un de l'autre, parmi H, des groupements $C_1$- à $C_{20}$-alcoxy à chaîne linéaire ou ramifiée, des groupements $C_1$- à $C_{20}$-alkyle à chaîne linéaire ou ramifiée ou des groupements $C_1$- à $C_{20}$-dialkylamino à chaîne linéaire ou ramifiée.

3. Utilisation selon la revendication 1 ou 2, **caractérisée en ce que** $R^2$, $R^6$ et $R^{13}$ désignent chacun H.

4. Utilisation selon l'une ou plusieurs parmi les revendications 1 à 3, **caractérisée en ce que** $R^3$, $R^5$, $R^9$, $R^{10}$ et $R^{12}$ désignent H.

5. Utilisation selon l'une ou plusieurs parmi les revendications 1 à 4, **caractérisée en ce que** $R^3$ ou $R^{12}$ désigne $SO_3H$ ou sulfonate.

6. Utilisation selon l'une ou plusieurs parmi les revendications 1 à 5, **caractérisée en ce que**
$R^4$ est choisi parmi H, des groupements $C_1$- à $C_4$-alcoxy ou des groupements $C_1$- à $C_4$-dialkylamino,
$R^{11}$ est choisi parmi des groupements $C_1$- à $C_6$-alkyle à chaîne linéaire ou ramifiée,
des groupements $C_1$- à $C_4$-dialkylamino,
des groupements $C_1$- à $C_4$-alcoxy.

7. Utilisation selon l'une ou plusieurs parmi les revendications 1 à 6, **caractérisée en ce que**
$R^6$ désigne $C_1$- à $C_{10}$-alcoxycarbonyle.

8. Utilisation d'au moins un composé de formule la, Ib, Ic ou Id selon l'une ou plusieurs parmi les revendications 1 à 7, **caractérisée en ce que** le au moins un composé est choisi parmi le 2-(hydroxyphényl-méthyl)-5-diéthylaminophénol, le 2-(hydroxyphénylméthyl)phénol, le 2-(hydroxyphénylméthyl)-5-sulfophénol, le 2-(hydroxyphénylméthyl)-5-méthoxy-4-sulfophénol, le 2-(hydroxyphénylméthyl)-5-méthoxyphénol, le 2-[(4-diéthylamino-2-hydroxyphényl)hydroxyméthyl]benzoate d'éthyle, le 2-[(4-diéthylamino-2-hydroxyphényl)hydroxyméthyl]-benzoate d'hexyle, le 2-[(4-diéthylamino-2-hydroxyphényl)hydroxy-méthyl]benzoate d'éthylhexyle, le 2-[(2-hydroxyphényl)hydroxy-méthyl]benzoate d'éthylhexyle, la 3-hydroxy-1,3-diphénylpropan-1-one, la 1-(4-tertio-butylphényl)-3-hydroxy-3-phénylpropan-1-one, la 1-(4-tertio-butylphényl)-3-hydroxy-3-(4-méthoxyphényl)propan-1-one, la 3-(4-tertio-butylphényl)-3-hydroxy-1-phénylpropan-1-one, la 3-(4-tertio-butylphényl)-3-hydroxy-1-(4-méthoxyphényl)propan-1-one ou la 3-(4-méthoxyphényl)-3-hydroxy-1 -(4-méthoxyphényl)propan-1-one.

9. Composés de formule Ib ou Ic

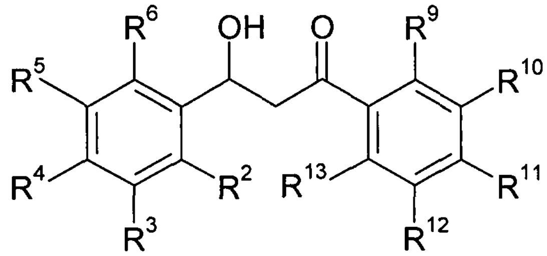

Ib

,

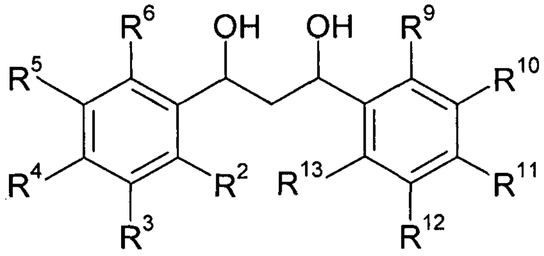

Ic

,

dans laquelle

$R^4$ et $R^{11}$ désignent chacun, indépendamment l'un de l'autre, H, un groupement $C_1$- à $C_{20}$-alkyle à chaîne linéaire ou ramifiée, un groupement $C_1$- à $C_{20}$-alcoxy à chaîne linéaire ou ramifiée ou un groupement $C_1$- à $C_{20}$-dialkylamino à chaîne linéaire ou ramifiée,
$R^6$ désigne H ou une fonction acide carboxylique, acide phosphorique, acide sulfonique, acide sulfurique ou sulfone, pouvant être estérifiée ou alkylée au moyen de groupements $C_1$- à $C_{20}$-alkyle à chaîne linéaire ou ramifiée ou de groupements $C_3$- à $C_{20}$-alcényle à chaîne linéaire ou ramifiée, et
$R^2$, $R^3$, $R^5$, $R^9$, $R^{10}$, $R^{12}$ et $R^{13}$ désignent H.

**10.** Composés selon la revendication 9, choisis dans le groupe constitué par

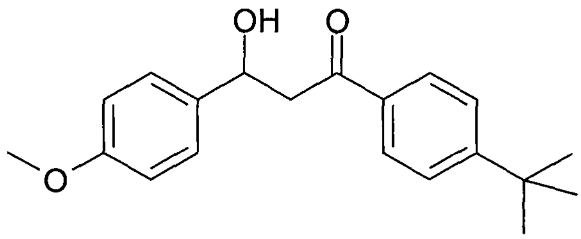

(1)

,

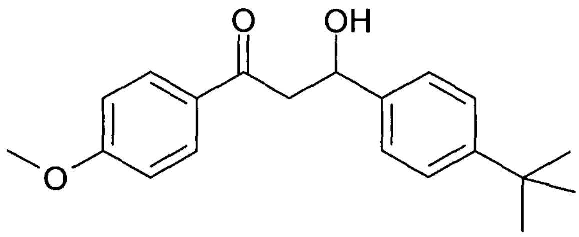

(2)

ou

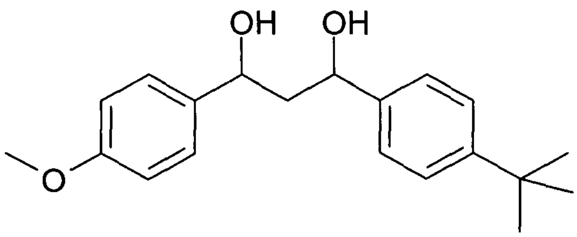

(3)

ou des sels des composés.

**11.** Composés de formule Ia, choisis parmi

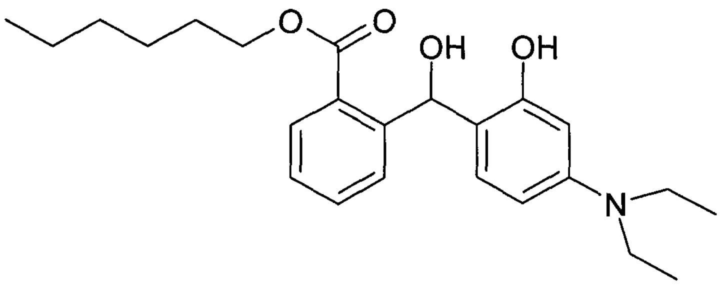

(4)

,

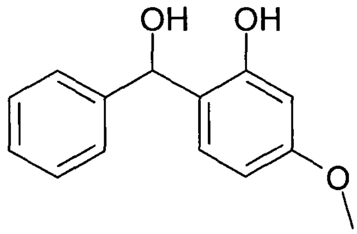

(5)

ou

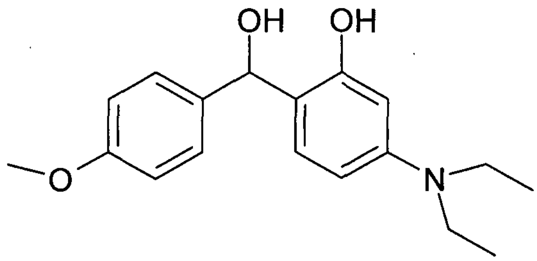

(6)

ou des sels des composés.

**12.** Composition comprenant au moins un véhicule qui est convenable pour des compositions cosmétiques ou pharmaceutiques, en particulier dermatologiques, des aliments ou compléments alimentaires ou des produits ménagers et au moins un composé de formules Ia à Id contenant des radicaux selon au moins l'une parmi les revendications 1 à 11.

**13.** Composition selon la revendication 12, **caractérisée en ce que** les compositions comprennent les un ou plusieurs composés de formules Ia à Id selon une quantité allant de 0,01 à 20% en poids.

**14.** Composition selon l'une ou plusieurs parmi les revendications 12 et 13, destinée à la protection des cellules de l'organisme contre un stress oxydatif, **caractérisée en ce qu'**elle comprend un(e) ou plusieurs autres antioxydants et/ou vitamines.

**15.** Composition selon l'une ou plusieurs parmi les revendications 12 à 14, **caractérisée en ce que** la composition comprend au moins un agent autobronzant.

**16.** Procédé de préparation d'une composition selon la revendication 12, **caractérisé en ce qu'**un composé de formules Ia à Id contenant des radicaux selon l'une ou plusieurs parmi les revendications 1 à 10, est mélangé avec un véhicule qui est convenable sur le plan cosmétique ou pharmaceutique ou pour des aliments ou des compléments alimentaires ou pour des produits ménagers.

**17.** Procédé de préparation d'un composé de formule Ib ou Ic

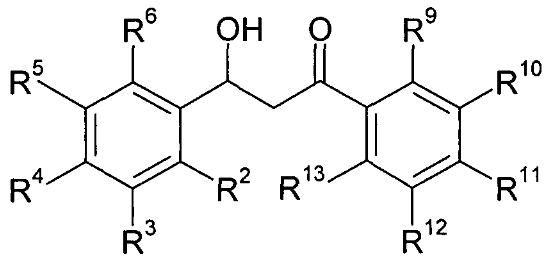

Ib

,

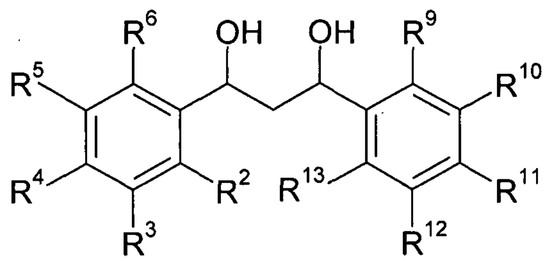

Ic

,

dans laquelle

$R^4$ et $R^{11}$ désignent chacun, indépendamment l'un de l'autre, H, un groupement $C_1$- à $C_{20}$-alkyle à chaîne linéaire ou ramifiée, un groupement $C_1$- à $C_{20}$-alcoxy à chaîne linéaire ou ramifiée ou un groupement $C_1$- à $C_{20}$-dialkylamino à chaîne linéaire ou ramifiée,
$R^6$ désigne H ou une fonction acide carboxylique, acide phosphorique, acide sulfonique, acide sulfurique ou sulfone, pouvant être estérifiée ou alkylée au moyen de groupements $C_1$- à $C_{20}$-alkyle à chaîne linéaire ou ramifiée ou de groupements $C_3$- à $C_{20}$-alcényle à chaîne linéaire ou ramifiée, et
$R^2$, $R^3$, $R^5$, $R^9$, $R^{10}$, $R^{12}$ et $R^{13}$ désignent H, **caractérisé en ce qu'**un composé de formule Ib en

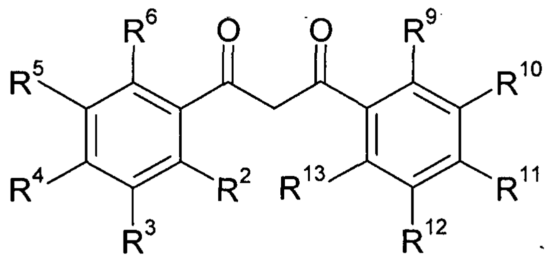

Ib en

,

dans laquelle les radicaux $R^2$ à $R^6$ et $R^9$ à $R^{13}$ revêtent une signification donnée ci-dessus, est hydrogéné.

**18.** Utilisation de composés de formule Ia, Ib, Ic ou Id selon la revendication 1, pour la protection de constituants de formulation sensibles à l'oxydation, tels que les colorants organiques ou inorganiques, les antioxydants, les vitamines, les composants de parfum, les composants huileux ou les constituants de matrice, tels que les émulsifiants, les épaississants, les agents filmogènes et les agents tensioactifs.

**19.** Composés de formule Ia, Ib, Ic ou Id selon la revendication 1, pour une utilisation dans le contrôle de la pigmentation, en particulier pour éclaircir des zones de peau.

Fig. 1

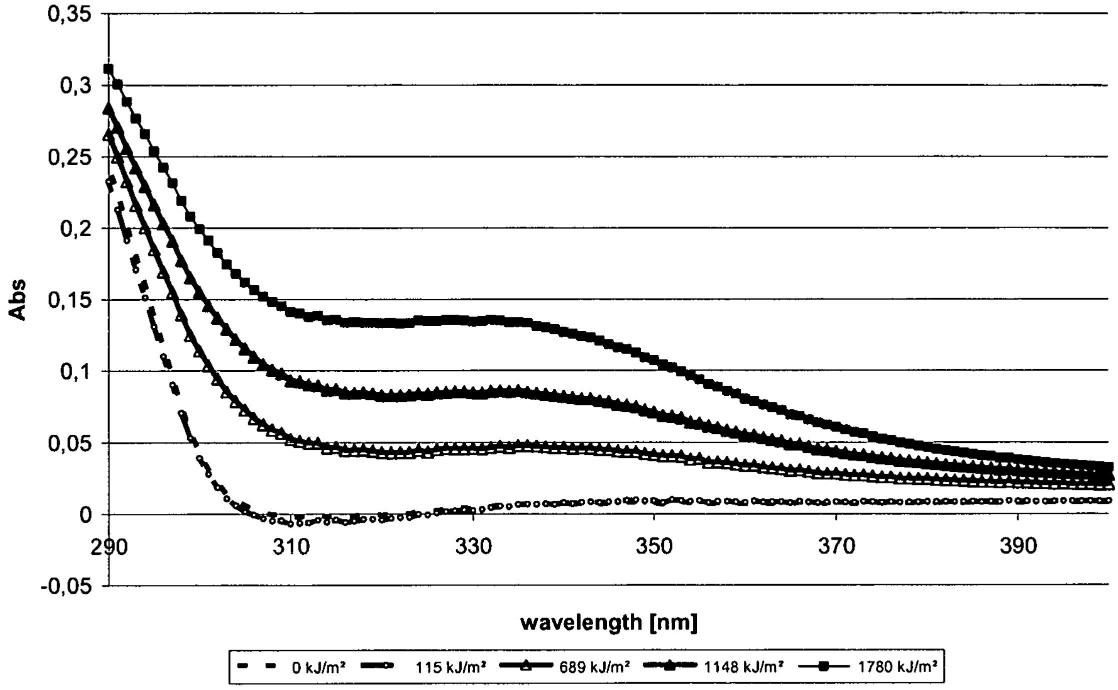
0,35
0,3
0,25
0,2
0,15
0,1
0,05
0
-0,05
Abs
290
310
330
350
370
390
wavelength [nm]
0 kJ/m²
115 kJ/m²
689 kJ/m²
1148 kJ/m²
1780 kJ/m²

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2006111233 A
- WO 2006111234 A
- DE 10232595 A
- US 6242099 B1
- WO 0009652 A
- WO 0072806 A
- WO 0071084 A
- EP 0671161 A
- DE 4116123 A


### In der Beschreibung aufgeführte Nicht-Patentliteratur


- **LAUT CD.** Römpp Chemie Lexikon - Version 1.0. Georg Thieme Verlag, 1995
- **C. SANCHEZ-MORENO ; J.A. LARRAURI ; F. SAURA-CALIXTO.** *J. Sci. Food Agric.,* 1998, vol. 76 (2), 270-276
- **E. A. GALINSKI et al.** *Eur. J. Biochem.,* 1985, vol. 149, 135-139
- **BÜNGER et al.** *International Journal of Cosmetic Science,* 2006, vol. 28, 135-146